# EUROPEAN PATENT SPECIFICATION

(11) **EP 2 717 926 B1**
(45) Date of publication and mention of the grant of the patent: **24.05.2017**
(21) Application number: 12800279.7
(22) Date of filing: 01.06.2012
(51) Int. Cl.: A61L 2/16, A61F 2/24, A61F 2/20, A61L 27/00, A61K 8/66, A61K 38/47, A01N 63/02

(54) **COMPOSITIONS AND METHODS TO PREVENT AND TREAT BIOFILMS**
ZUSAMMENSETZUNGEN UND VERFAHREN ZUR PRÄVENTION UND BEHANDLUNG VON BIOFILMEN
COMPOSITIONS ET PROCÉDÉS POUR PRÉVENIR ET TRAITER DES BIOFILMS

(30) Priority: 13.06.2011 US 201161520654 P; 26.05.2012 US 201213481787
(43) Date of publication of application: 16.04.2014
(73) Proprietor: Ivanova, Svetlana A., Winter Springs, Florida 32708 (US)
(72) Inventor: DAVIS, Dennis W., Mt. Dora, Florida 32757 (US); ARENZ, Brad W., Orlando, Florida 32812 (US); CONNELLAN, Thomas K., Orlando, Florida 32819 (US); IVANOVA, Svetlana A., Winter Springs, Florida 32708 (US)
(74) Representative: Hocking, Adrian Niall
(86) International application number: PCT/US2012/040408
(87) International publication number: WO 2012/173796

(56) References cited:
- EP-A1- 1 068 871
- EP-A1- 1 081 232
- EP-B1- 0 820 516
- WO-A2-97/42326
- WO-A2-2006/032011
- JP-A- H0 751 063
- JP-A- 2003 061 651
- US-A1- 2006 121 019
- US-A1- 2009 202 516
- US-A1- 2011 129 454
- US-B1- 6 759 040
- E. W. Petzold ET AL: "Characterization and Regulation of the Trehalose Synthesis Pathway and Its Importance in the Pathogenicity of Cryptococcus neoformans", Infection and Immunity, vol. 74, no. 10, 20 September 2006 (2006-09-20), pages 5877-5887, XP055340600, US ISSN: 0019-9567, DOI: 10.1128/IAI.00624-06

## Description

### I. Cross Reference to Related Applications

This application claims the benefit of U.S. Provisional Application No. 61/520,654 filed June 13, 2011. The disclosure of the provisional application is incorporated herein by reference.

### II. Field

The present disclosure is generally related to compositions and methods to prevent and treat biofilms.

### III. Description of Related Art

Over the last century, bacterial biofilms have been described as a ubiquitous form of microbial life in various ecosystems which can occur at solid-liquid, solid-air, liquid-liquid, and liquid-air interfaces. The general theory of biofilm predominance was defined and published in 1978 (Costerton JW, Geesey GG, and Cheng GK, "How bacteria stick," Sci. Am., 1978; 238: 86-95.). The basic data for this theory initially came mostly from natural aquatic ecosystems showing that more than 99.9% of the bacteria grow in biofilms on a variety of surfaces, causing serious problems in industrial water systems as well as in various pipelines and vessels.

Later this fundamental theory of bacterial biofilm was accepted in the medical and dental areas. New and advanced methods for the direct examination of various biofilms showed that microorganisms that cause many medical device-related and other chronic infections in the human body actually grow in biofilms in or on these devices, as well as on mucosal linings of various organs and systems (oral cavity, respiratory tract, eyes, ears, GI tract, and urinary tract). As stated in this theory, "bacteria have certain basic survival strategies that they employ wherever they are" (Donlan RM and Costerton JW, "Biofilms: Survival Mechanisms of Clinically Relevant Microorganisms," Clinical Microbiology Reviews, Apr. 2002: 167-193.)

### The Nature and Structure of Biofilms

Over decades, direct physical and chemical studies of various biofilms (mostly grown in laboratory settings) show that they consist of single microbial cells and microcolonies, all embedded in a highly hydrated exopolymer matrix comprising biopolymers of microbial origin, such as polysaccharides (the major component), proteins, glycoproteins, nucleic acids, lipids, phospholipids, and humic substances; ramifying water channels bisect the whole structure, carrying bulk fluid into the biofilm by convective flow, providing transport of nutrients and waste products, and contributing to a pH gradient within the biofilm (Costerton JW and Irvin RT, "The Bacteria Glycocalyx in Nature and Disease," Ann. Rev. Microbiol., 1981; 35: 299-324.); (de Beer D, Stoodley P, and Lewandowski Z, "Liquid flow in heterogeneous biofilms," Biotechnol. Bioeng, 1994; 44: 636-641.); (Himmelsbach DS and Akin DE, "Near-Infrared Fourier-Transform Raman Spectroscopy of Flax (Linum usitatissimum L.) Stems," J Agric Food Chem, 1998; 46: 991-998.); (Maquelin K, Kirschner C, Choo-Smith LP, van den Braak N, Endtz HP, Naumann D, and Puppels GJ, "Identification of medically relevant microorganisms by vibrational spectroscopy," J Microbiol Methods, 2002; 51: 255-271.); (Neu TR and Marshall KC, "Bacterial Polymers: Physicochemical Aspects of Their Interactions at Interfaces," J Biomater Appl, 1990; 5: 107-133.); (Neugebauer U, Schmid U, Baumann K, Ziebuhr W, Kozitskaya S, Deckert V, Schmitt M, Popp J, "Toward a Detailed Understanding of Bacterial Metabolism - Spectroscopic Characterization of Staphylococcus Epidermidis," ChemPhysChem, 2007; 8: 124-137.); (Weldon MK, Zhelyaskov VR, Morris MD, "Surface-enhanced Raman spectroscopy of lipids on silver microprobes," Appl Spectrosc, 1998; 52: 265-269.). Depending on the biofilm type and the microorganisms involved, microcolonies of microbial cells make up approximately 10% - 15% of the biofilm by volume, and the biofilm matrix comprises approximately 85% - 90%. Water, the major component of the biofilm matrix, can make up to 95% - 98% of the matrix volume, and the particulate fraction of the matrix can comprise the rest 2% - 5% correspondingly. Extracellular polysaccharides and proteins have been considered to be the key components of the biofilm matrix and have been most extensively studied over decades (Sutherland IW, "The biofilm matrix - an immobilized but dynamic microbial environment," Trends Microbiol, 2001; 9: 222-227.); (Stewart PS and Costerton JW, "Antibiotic resistance of bacteria in biofilms," Lancet, 2001; 358: 135-138.); (Staudt C, Horn H, Hempel DC, Neu TR, "Volumetric measurements of bacteria and EPS-glycoconjugates in biofilms," Biotechnol Bioeng, 2004; 88: 585-592.); (Zhang XQ, Bishop PL, and Kupferle MJ, "Measurement of polysaccharides and proteins in biofilm extracellular polymers," Water Sci Technol, 1998; 37: 345-348.).

Polysaccharides, postulated to be the key component of the biofilm matrix, provide diverse structural variations of the glycocalux formed by saprophytic and pathogenic microorganisms in a variety of environments (Barbara Vu, et al., "Review. Bacterial extracellular polysaccharides involved in biofilm formation," Molecules, 2009; 14: 2535- 2554; doi: 3390/molecule 14072535.). The types of polysaccharides in microbial biofilms are of enormous range and depend on the genetic profile of microorganisms involved and the physicochemical properties of local environment (Sutherland IW, "The biofilm matrix - an immobilized but dynamic microbial environment," Trends Microbiol., 2001; 9: 222-227.). Many polysaccharides are constitutively produced by various bacteria as structural elements of the bacterial cell wall and virulence factors; they can stay attached to the bacterial cell wall surface, forming a complex network surrounding the cell with electrostatic and hydrogen bonds involved, or they can be released into media as exopolysaccharides (EPS) (Mayer C, Moritz R., Kirschner C., Borchar W, Maibaum R, Wingender J, and Hemming HC, "The role of intermolecular interactions: studies on model systems for bacterial biofilms," Int J Biol Macromol, 1999; 26: 3-16.). Polysaccharides, as well as mono- and disaccharides, can be taken by bacteria from the environment and metabolized as a carbon source, and their metabolism is genetically regulated via balanced production of enzymes for both synthesis and degradation pathways (Sutherland IW, "Polysaccharases for microbial polysaccharides," Carbohydr Polym, 1999; 38: 319-328.). Depending on their structure, EPS can bind various amount of water, and some of them (such as cellulose, mutan or curdlan) can even exclude most water from their tertiary structure. Over the years, the gel-like viscosity of the biofilm matrix was attributed mainly to the physical and chemical properties of the polysaccharides involved (Christensen BE, "The role of extracellular polysaccharides in biofilms," J Biotechnol, 1989; 10: 181-202.); (Stoodley P, et al., "Oscillation characteristics of biofilm streamers in turbulent flowing water as related to drag and pressure drop," Biotechnol Bioeng, 1998; 57: 536- 544.). Exopolysaccharides can be neutral homopolymers (such as cellulose, dextrans, levans), but the majority are polyanionic (for example, alginates, gellan, xanthan produced by Gram-negative bacteria) with attraction of divalent cations (Ca, Mg) to increase binding force, and a few are polycationic, such as those produced by some Gram-positive bacteria (Sutherland IW, "Biotechnology of Exopolysaccharides," Cambridge: Cambridge University Press, 1990.); (Mack D, Fische W, Krokotsc A, Leopold K, Hartmann R, Egge H, and Laufs R, " The intercellular adhesin involved in biofilm accumulation of Staphylococcus epidermidis is a linear β-1, 6-linked glucosaminoglycan: purification and structural analysis," J Bacteriol, 1996; 178: 175-183.).

Because only small amounts of the biofilm-derived EPS are normally available for direct studies, the researchers usually use data derived from planktonic cell cultures and extrapolate them to biofilms. There is no conclusive evidence to support the idea of existence of the biofilm-specific polysaccharides, and to date, all studied polysaccharides present in various biofilms resemble closely the corresponding polymers synthesized by planktonic cells. It has been proposed that the increased amount of polysaccharides in biofilm (one or more, specific for a given bacteria in any given biofilm) can be part of a stress response in biofilm-grown microorganisms, and bacteria form exopolysaccharides as a by-product to release reducing equivalents accumulated in non-optimal growth conditions (Creti R, Koch S, Fabretti F, Baldassarri L, and Huebneri J, "Enterococcal colonization of the gastro-intestinal tract: role of biofilm and environmental oligosaccharides," BMC Microbiology, 2006; 6: 60 doi: 10.1186/1471-2180-6-60.); (Rinker KD, Kelly RM, "Effect of carbon and nitrogen sources on growth dynamics and exopolysaccharide production for the hyperthermophilic archaeon Thermococcus litoralis and bacterium Thermotoga maritime," Biotechnol Bioeng, 2000; 69: 537-547.); (Sutherland IW, "Biofilm exopolysaccharides: a strong and sticky framework," Microbiology, 2001; 147: 3-9.).

Other extracellular products (specific substances or by-products of bacterial metabolism), as well as detritus, can be either released into the biofilm from aging and lysed cells or trapped within the biofilm matrix, and "cemented" there by mixture of exopolysaccharides (Christensen BE, "The role of extracellular polysaccharides in biofilms," J. Biotechnol., 1989; 10: 181-201.). These extracellular products include small sugars (mono-, disaccharides), polyols, proteins, glycoproteins, enzymes, lipids, glycolipids, phospholipids, nucleic acids, and DNA (Boyd A and Chakrabarty AM, "Role of alginate lyase in cell detachment of Pseudomonas aeruginosa," Appl Environ Microbiol, 1994; 60: 2355-2359.); (Harz M, Røsch P, Peschke KD, Ronneberger O, Burkhardt H, and Popp J, "Micro-Raman spectroscopic identification of bacterial cells of the genus Staphylococcus and dependence on their cultivation conditions," Analyst, 2005; 130: 1543-1550.); (Nottingher I, Verrier S, Haque S, Polak JM, Hench LL, "Spectroscopic study of human lung epithelial cells (A549) in culture: living cells versus dead cells," Biopolymers, 2003; 72: 230-240.); (Sutherland IW, "A natural terrestrial biofilm," J Ind Microbiol, 1996; 17: 281-283.); (Webb JS et al, "Cell death in Pseudomonas aeruginosa biofilm development," J. Bacteriol., 2003; 185: 4585-4592.); (Weldon MK, Zhelyaskov VR, Morris MD, "Surface-enhanced Raman spectroscopy of lipids on silver microprobes," Appl Spectrosc, 1998; 52: 265-269.); (Yarwood JM, et al., "Quorum sensing in Staphylococcus aureus biofilms," J. Bacteriol., 2004; 186: 1838-1850.). It has been suggested that extracellular DNA, released from the lysed cells, plays an important role in supporting the biofilm structure and provides opportunities for microorganisms to exchange the genetic material for possible development of the biofilm-specific phenotypes (Costerton JW, Veeh R, Shirtliff M, Pasmore M, Post C, and Ehrich GD, "The application of biofilm science to the study and control of chronic bacterial infections," J. Clin. Invest., 2003; 112: 1466-1477.); (Gilbert P, Maira-Litran T, McBain AJ, Rickard AH, and Whyte FW, "The physiology and collective recalcitrance of microbial biofilm communities," Adv. Microb. Physiol., 2002; 46: 202-256.); (Osterreicher-Ravid D, Ron EZ, &Rosenberg E, "Horizontal transfer of an exopolymer complex from one bacterial species to another," Environ Microbiol, 2000; 2: 366-372.); (Stoodley P, Sauer K, Davies DG, and Costerton JW, "Biofilms as complex differentiated communities," Annu. Rev. Microbiol., 2002; 56: 187-209.); (Whitchurch CB, et al., "Extracellular DNA required for bacterial biofilm formation," Science, 2002; 295: 1487.).

It has been proposed that in the dynamic environment of biofilm, microorganisms use special chemical signaling molecules to communicate (the process called quorum-sensing - QS), and the presence of an adequate number of neighboring cells with coordinated chemical signaling between them allow bacteria to properly respond to changes in environmental conditions, including insult from antimicrobials, and benefit from living in the biofilm community. It was assumed that QS can regulate extracellular polysaccharide production, based on the major alterations in the extracellular matrix of laboratory-grown Pseudomonas aeruginosa biofilm when the mutant strain was unable to produce the N-(3-oxododecanoyl)-L-homoserine lactone signal specific for QS (Davies D, Parsek M, Pearson J, et al., "The involvement of cell-to-cell signals in the development of a bacterial biofilm," Science, 1998; 280: 295-298.); (Singh P, Schaeffer A, Parsek M, et al., "Quorum sensing signals indicate that cystic fibrosis lungs are infected with bacterial biofilms," Nature, 2000; 407: 762-764.). But to date, the quorum-sensing-regulated genes involved in Pseudomonas aeruginosa biofilm matrix production have not been identified, and the pel and/or psl genes (regulating production of other polysaccharides PEL and PSL) have not been revealed as quorum-sensing-regulated genes as well (Branda SS, Vik A, Friedman L, and Kolter R, "Biofilms: the matrix revisited," Trends in Microbiology, 2005; 13(1): 20-26.); (Whiteley M, et al., "Identification of genes controlled by quorum sensing in Pseudomonas aeruginosa," Proc. Natl. Acad. Sci. U.S.A., 1999; 96: 13904-13909.). Also, the role of quorum sensing in resistance of biofilm to antimicrobials is not clear yet; for example, the laboratory mutants defective in quorum sensing, are unaffected in their resistance to detergents and antibiotics (Brooun A, et al., "A dose-response study of antibiotic resistance in Pseudomonas aeruginosa biofilms," Antimicrob. Agents Chemother, 2000; 44: 640-646.).

According to a classical model, any biofilm can be described as: a non-homogenous multi-layer structure with dynamic environment; growing in a 3-dimensional mode, with constant addition of the new layers and detachment of the parts of the biofilm; with spatial and temporal heterogeneity within the biofilm and variations in bacterial growth rate; with different metabolic and genetic activities of the microorganisms resulting in increased resistance to antimicrobials (including antibiotics) and host defense mechanisms (Charaklis WG, Marshall KC , "Biofilm as a basis for interdisciplinary approach," pp.3-15, In: Biofilms, 1990, John Wiley and Sons, Charaklis WG. and Marshall KC. (ed.), New York, N.Y.); (Fux CA, et al., "Review. Survival strategies of infectious biofilms", Trends in Microbiology, January 2005; Vol. 13, No 1: 34-40.). The heterogeneity within the biofilm has been confirmed for protein synthesis and respiratory activity, but the DNA content remained relatively constant throughout biofilm (Wentland EJ, et al., "Spatial variations in growth rate within Klebsiella pneumoniae colonies and biofilm," Biotechnol. Prog., 1996; 12: 316-321.); (Xu KD, et al., "Biofilm resistance to antimicrobial agents," Microbiology, 2000; 146: 547-549.). An oxygen tension gradient exists within biofilm with the superficial areas being more metabolically active than the deeper areas where bacteria adapt to decreased oxygen availability (De Beer D, Stoodley P, Roe F, et al., "Effects of biofilm structure on oxygen distribution and mass transport," Biotechnology Bioengineering, 1994; 43: 1131-1138.). The outer layers of biofilm are more permeable to antimicrobials due to slow build-up of polysaccharides and other constituents (proteins, lipids, etc.), and the inner (deeper) layers are more dense, compressed, and less permeable. Bacteria in the outer layers of biofilm, exposed to the bulk medium, grow faster and can be less resistant to antimicrobials. Conversely, the bacteria in the inner or deeper layers, located closer to the attached surface, grow slower, adapting to decreased oxygen and nutrients availability, and in time, can become more resistant to antimicrobials with possible consequent emergence of biofilm-specific antibiotic-resistant phenotype (Brown MR, et al., "Resistance of bacterial biofilms to antibiotics: a growth-rate related effect?," J. Antimicrob. Chemother., 1998; 22: 777-780.).

It has been proposed that "any given cell within the biofilm will experience a slightly different environment compared with other cells within the same biofilm, and thus be growing at a different rate" (Mah TC, and O' Toole GA, "Review. Mechanisms of biofilm resistance to antimicrobial agents," Trends in Microbiology, Jan. 2001, 9(1): 34-39.). With continuous bacterial growth, increased cell density triggers the general stress response in microbial cells, as confirmed by increased production of osmoprotectant trehalose and degrading enzyme catalase, with higher concentration of trehalose in proximity to the pathogenic cell colonies (Liu X, et al., "Global adaptations resulting from high population densities in Escherichia coli cultures," J. Bacteriol., 2000; 182: 4158- 4164.). These events result in physiological changes in biofilm, including reduced flow of solutes (nutrients) into biofilm and diminished growth rate of bacterial microcolonies for genotype survival (Brown M R, and Barker J, "Unexplored reservoirs of pathogenic bacteria: protozoa and biofilms," Trends Microbiol., 1999; 7: 46-50.); (Mah TC., and O' Toole GA, "Review: Mechanisms of biofilm resistance to antimicrobial agents", Trends in Microbiology, Jan. 2001; 9(1): 34-39.).

About two decades ago, the existence of biofilm-specific phenotypes of bacteria was an emerging idea. Such biofilm-specific phenotypes, thought to be induced in a subpopulation of microorganisms upon attachment to a surface, were proposed to express specific biofilm-related genes compared with their planktonic counterparts (Kuchma SL, and O'Toole G A, "Surface-induced and biofilm-induced changes in gene expression," Curr. Opin. Biotechnol., 2000; 11: 429-433.). Multiple research data, based mostly upon the genetic studies of the laboratory-constructed and laboratory-grown mutant strains, provided supportive evidence that the biofilm-grown cells differ from their planktonic counterparts in specific properties, including nutrients utilization, growth rate, stress response, and increased resistance to antimicrobial agents and the host defenses.

### Biofilm Resistance to Antimicrobial Agents

The mechanism of resistance to antimicrobial agents (including antibiotics) in biofilm-related microorganisms is different from plasmid, transposons, and mutations that confer innate resistance in individual bacterial cells (Stewart PS and Costerton JW, "Review. Antibiotic resistance of bacteria in biofilms," Lancet, 2001; 358: 135-138.); (Costerton JW, Stewart PS, and Greenberg E, "Bacterial biofilms: a common cause of persistent infections," Science, 1999; 284: 1318-1322.); (Costerton JW and (Stewart PS, "Biofilms and device-related infections," In: Nataro JP, Blaser MJ, Cunningham-Rundles S., (eds.), Persistent bacterial infections. Washington, DC: ASM Press, 2000; 432- 439.).

Multiple research studies provided basis for various mechanisms of biofilm resistance to antimicrobials, including:
- physical and/or chemical diffusion barriers to penetration of antimicrobials and host defense cells into the exopolymer matrix of biofilm
- activation of a general stress response of the microorganisms
- slow growth of the microorganisms
- possible emergence of a biofilm-specific bacterial phenotype

These mechanisms can be applied to any type of biofilm, varying with the bacteria present and the type of antimicrobials being used (Geddes A, "Infection in the twenty-first century: Predictions and postulates," J Antimicrob Chemother, 2000; 46: 873-878.); (Stewart PS, "Theoretical aspects of antibiotic diffusion into microbial biofilms," Antimicrob. Agents Chemother., 1996; 40: 2517- 2522.); (Stewart PS, "Mechanisms of antibiotic resistance in bacterial biofilms," Int J Med Microbial, 2002; 292: 107-113.).

Most of the biofilm-resistance mechanisms are provided by the biofilm exopolymer matrix as the initial physical and/or chemical barrier that can prevent, inhibit or delay penetration of antimicrobials and host defense cells into the biofilm. The diffusion of antimicrobials through the biofilm can be inhibited by various means: for example, the common disinfectant chlorine is consumed by chemical reaction within the matrix of a mixed Klebsiella pneumoniae and Pseudomonas aeruginosa biofilm (de Beer D, et al., "Direct measurement of chlorine penetration into biofilms during disinfection," Appl. Environ. Microbiol., 1994; 60: 4339- 4344.); antibiotic ciprofloxacin binds to the biofilm components (Suci PA, et al., "Investigation of ciprofloxacin penetration into Pseudomonas aeruginosa biofilms," Antimicrob Agents Chemother, 1994; 38: 2125-2133.); Pseudomonas aeruginosa biofilm prevents diffusion of piperacillin (Hoyle B, et al., "Pseudomonas aeruginosa biofilm as a diffusion barrier to piperacillin," Antimicrob. Agents Chemother., 1992: 36: 2054- 2056.); positively charged aminoglycosides bind to negatively charged matrix polymers, such as β 1,4-glucosaminoglycan in Staphylococcus epidermidis biofilm and alginate in Pseudomonas aeruginosa biofilm (Lewis K, " Riddle of biofilm resistance," Antimicrob Agents Chemother., 2001; 45: 999-1007.); (Walters MC, et al., "Contributions of antibiotic penetration, oxygen limitation, and low metabolic activity to tolerance of Pseudomonas aeruginosa biofilms to ciprofloxacin and tobramycin," Antimicrob. Agents Chemother., 2003; 47: 317-323.); (Gordon CA, Hodges NA, Marriott C, " Antibiotic interaction and diffusion through alginate exopolysaccharide of Cystic fibrosis - derived Pseudomonas aeruginosa," J. Antimicrob. Chemother., 1988; 22: 667- 674.); (Nichols WW, et al., "Inhibition of tobramycin diffusion by binding to alginate," Antimicrob. Agents Chemother., 1988; 32: 518- 523.); the additional matrix component colanic acid, produced by mucoid phenotype of E. coli, supports biofilm maturation and provides a thicker biofilm (Danese PN, et al., "Exopolysaccharide production is required for development of Escherichia coli K-12 biofilm architecture," J. Bacteriol., 2000; 182: 3593- 3596.); penetration of antifungal agent nystatin into the mycelium of Aspergillus fumigatus submerged in medium and covered by thin layer of exopolymer matrix is higher than into the aerial-grown colony covered by thick layer of extracellular matrix (Beauvais A, et al., "An extracellular matrix glues together the aerial-grown hyphae of Aspergillus fumigatus," Cellular Microbiology, 2007; 9 (6): 1588-1600.); secreted IgG antibodies fail to penetrate biofilm because of matrix binding (de Beer D, et al., "Measurement of local diffusion coefficients in biofilms by micro-injection and confocal microscopy," Biotechnol. Bioeng., 1997; 53: 151- 158.); alginate produced by mucoid phenotype of Pseudomonas aeruginosa protects bacteria from phagocytosis by host leukocytes and INF-γ activated macrophages (Bayer AS, et al., "Functional role of mucoid exopolysaccharide (alginate) in antibiotic-induced and polymorphonuclear leukocyte-mediated killing of Pseudomonas aeruginosa," Infect. Immun., 1991; 59: 302- 308.); (Leid JG, Willson CJ, Shirtliff ME, Hassett DJ, Parsek MR, and Jeffers AK, "The exopolysaccharide alginate protects Pseudomonas aeruginosa biofilm bacteria from IFN-gamma-mediated macrophage killing." J Immunol, 2005; 175: 7512-7518.).

Antimicrobials diffusion can also be inhibited or delayed by specific active substances produced by bacteria themselves: for example, enzyme catalase produced by Pseudomonas aeruginosa spp. degrades hydrogen peroxide on diffusion into thick biofilm (Stewart PS, et al., "Effect of catalase on hydrogen peroxide penetration into Pseudomonas aeruginosa biofilms," Appl. Environ. Microbiol., 2000; 66: 836- 838.); ampicillin is unable to penetrate biofilm of Klebsiella pneumoniae due to ampicillin-degrading enzyme Beta-lactamase (Anderi JN, et al., "Role of antibiotic penetration limitation in Klebsiella pneumoniae biofilm resistance to ampicillin and ciprofloxacin," Antimicrob. Agents Chemother., 2000; 44: 1818-1824.); (Bagge N, Hentzer M, Andersen JB, Ciofu O, Givskov M, and Høiby N, "Dynamics and spatial distribution of beta-lactamase expression in Pseudomonas aeruginosa biofilms," Antimicrob Agents Chemother, 2004; 48: 1168-1174.); extracellular slime derived from coagulase-negative Staphylococci reduces the effect of glycopeptide antibiotics (Konig C, et al., "Factors compromising antibiotic activity against biofilms of Staphylococcus epidermidis," Eur. J. Clin. Microbiol. Infect. Dis., 2001; 20: 20-26.); (Souli M and Giamarellou H., "Effects of slime produced by clinical isolates of coagulase-negative staphylococci on activities of various antimicrobial agents," Antimicrob. Agents Chemother., 1998; 42: 939- 941.); a PMN toxin, rhamnolipid B, produced by Pseudomonas aeruginosa is known to kill neutrophils (Jensen PØ, Bjarnsholt T, Phipps R, Rasmussen TB, Calum H, Christoffersen L, et al., "Rapid necrotic killing of polymorphonuclear leukocytes is caused by quorum-sensing-controlled production of rhamnolipid by Pseudomonas aeruginosa," Microbiology, 2007; 153: 1329-1338.).

Delayed penetration of antimicrobials into the biofilm can provide enough time for bacteria to induce the expression of various genes regulating the stress response and mediating resistance to antimicrobials (Jefferson KK, Goldmann DA, and Pier GB, "Use of confocal microscopy to analyze the rate of vancomycin penetration through Staphylococcus aureus biofilms," Antimicrob Agents Chemother, 2005; 49: 2467-2473.); (Anwar H, Strap JL, and Costerton JW, "Establishment of aging biofilms: a possible mechanism of bacterial resistance to antimicrobial therapy," Antimicrob Agents Chemother, 1992; 36: 1347-1351.). The central regulator of a general stress response is the alternate sigma-factor RpoS induced by high cell density, and the presence of activated gene rpoS' mRNA was detected by RT-PCR in sputum from Cystic Fibrosis patients with chronic Pseudomonas aeruginosa biofilm infections (Foley I, et al., "General stress response master regulator rpoS is expressed in human infection: a possible role in chronicity," J. Antimicrob. Chemother., 1999; 43: 164-165.). Also, it has been shown that an additional sigma-factor Alg acted in concert with RpoS to control general stress response in laboratory grown Pseudomonas aeruginosa during biofilm formation and maturation, and several other genes were upregulated as well, including algC (controlling phosphomannomutase, involved in exopolysaccharide alginate synthesis), algD, algU, and genes controlling palyphasphakinase synthesis (Davis DG and Geesey GG, "Regulation of the alginate biosynsthesis gene algC in Pseudomonas aeruginosa during biofilm development in continuous culture," Appl. Environ. Microbiol., 1995; 61: 860-867.). It has been demonstrated that as many as 45 genes differed in expression between sessile cells and their planktonic counterparts during the biofilm development in laboratory settings.

### Biofilm-based Medical Conditions and Diseases

Comprehensive review of the biofilm-based human infections as well as the biofilms on medical devices was published by Rodney M. Donlan and J. William Costerton (Donlan RM and Costerton JW, "Review. Biofilms: Survival mechanisms of clinically relevant microorganisms," Clinical Microbiology Reviews, Apr. 2002; 167-193.). Microbial biofilms are important factors in the pathogenesis of various human chronic infections, including native valve endocarditis (NVE), line sepsis, chronic otitis media, chronic sinusitis and rhinosinusitis, chronic bronchitis, cystic fibrosis pseudomonas pneumonia, chronic bacterial prostatitis, chronic urinary tract infections (UTIs), periodontal disease, chronic wound infections, osteomyelitis (Costerton JW, Stewart P, Greenberg E, "Bacterial biofilms: a common cause of persistent infections," Science, 1999; 284: 1318-1322.); (Hall-Stoodley L and Stoodley P, "Evolving concepts in biofilm infections," Cellular Microbiology, 2009; 11 (7): 1034-1043.). Microbial biofilms are detected on various medical devices (prosthetic heart valves, central venous catheters, urinary catheters, contact lenses, tympanostomy tubes, intrauterine devices), as well as on medical equipment (endoscopes, dialysis systems, nebulizers, dental unit water lines), and on a variety of surfaces in hospitals and other medical settings (Costeron JW and Stewart PS, "Biofilms and device-related infections," In: Nataro J.P., Blaser M.J., Cunningham-Rundles S., eds. Persistent bacterial infections. Washington, DC: ASM Press, 2000; 432-439.); (Bryers JD, "Medical Biofilms," Biotechnology and Bioengineering, 2008; 100 (1) May 1.). Due to their specific features, chronic biofilm-based infections require different interventional approaches for effective treatment (Stewart PS and Costerton JW., "Review. Antibiotic resistance of bacteria in biofilms," Lancet, 2001; 358: 135-138.); (Donlan RM and Costerton JW, "Review. Biofilms: Survival mechanisms of clinically relevant microorganisms," Clinical Microbiology Reviews, Apr. 2002; 167-193.); (Costerton JW, Stewart PS, and Greenberg EP, "Bacterial biofilms: a common cause of persistent infections," Science, 1999; 284: 1318-1322.); (Costerton JW and Stewart PS, "Biofilms and device-related infections," In: Nataro JP, Blaser MJ, Cunningham-Rundles S, eds. Persistent bacterial infections. Washington, DC: ASM Press, 2000; 432- 439.); (Wolcott RD, M.D. and Ehrlich GD, Ph.D., "Biofilms and chronic infections," JAMA, 2008, Vol. 299, No 22.); (Costerton JW, Irvin RT, "The Bacteria Glycocalyx in Nature and Disease," Ann. Rev. Microbiol., 1981; 35: 299-324.); (Costerton JW, et al., "The application of biofilm science to the study and control of chronic bacterial infections," J. Clin. Invest., 2003; 112: 1466-1477.).

### Native Valve Endocarditis

The development of Native Valve Endocarditis (NVE) results from the interaction between the endothelium of the heart (generally, of the mitral, aortic, tricuspid, and pulmonic valves) and microorganisms circulating in the bloodstream (Livornese LL and Korzeniowski OM, "Pathogenesis of infective endocarditis," pp. 19-35. In: Infective endocarditis, Kaye D. (ed.), 2-nd ed., 1992; Raven Press, New York, N.Y.). Microorganisms usually do not adhere to intact endothelium. There should be contributing factors that promote adherence, such as: damaged endothelium (as in vasculitis), formation of initial thrombotic lesions of heart valves (as in nonbacterial thrombotic endocarditis - NBTE), accumulation of fibronectin secreted by endothelial cells, platelets and fibroblasts in response to vascular injury, which can simultaneously bind to fibrin, collagen, human cells, and bacteria, specific fibronectin receptors in some bacteria (Streptococcus sanguis, Staphylococcus aureus), high-molecular weight dextrans produced by various Streptococci that promote adherence to the surface of the thrombus in NBTE (Lowrance JH, Baddour LM, and Simpson WA, "The role of fibronectin binding on the rate model of experimental endocarditis caused by Streptococcus sanguis," J. Clin. Investig. 86: 7-13.); (Roberts RB, "Streptococcal endocarditis: the viridins and beta hemolytic streptococci," pp. 191-208. In: Infective endocarditis, Kaye D. (ed.), 2-nd ed., 1992; Raven Press, New York, N.Y.). The most metabolically active colonies were detected on the surface of the thrombus, forming initial biofilm there (Durack DT and Beeson PB, "Experimental bacterial endocarditis II. Survival of bacteria in endocardial vegetations," Br. J. Pathol., 1972, 53: 50-53.). Clinical research of 2345 cases of NVE demonstrated a variety of microorganisms involved: Streptococci (including Streptococcus viridans, Streptococcus bovis), Enterococci, Pneumococci ∼ in 56% of cases; Staphylococci ∼ in 25% of cases (∼19% - Coagulase positive and ∼6% - Coagulase negative); Gram-negative bacteria ∼ in 11% of cases, and Fungi (Candida and Aspergillus spp.) ∼ in 10% of cases; all these microorganisms gained access to the bloodstream primarily via the oropharynx, gastrointestinal tract, and genitourinary tract (Tunkel AR and Mandell GI, " Infecting microorganisms," pp. 85-97. In: Infective endocarditis, Kaye D. (ed.), 2-nd ed., 1992; Raven Press, New York, N.Y.).

### Biofilm-based Chronic Infections in the Respiratory Tract

In the upper respiratory tract, bacterial biofilms have been demonstrated in chronic tonsillitis, chronic adenoiditis, chronic sinusitis and chronic rhinosinusitis (CRS), chronic otitis media (OM), and cholesteatoma. In clinical specimens from patients with chronic and recurrent tonsillitis, both attached and aggregated biofilm-associated bacteria were detected in mucosal epithelium of tonsils removed for chronic tonsillitis (in 73% of cases) and in 75% of cases of tonsils removed due to hypertrophy alone (Chole RA and Faddis BT, "Anatomical evidence of microbial biofilms in tonsillar tissues: a possible mechanism to explain chronicity," Arch Otolaryngol Head Neck Surg, 2003; 129: 634-636.). Microbial biofilms associated with epithelial lining with presence of a carbohydrate matrix in situ were demonstrated in clinical specimens of human adenoids removed for chronic adenoiditis (Kania RE, Lamers GE, Vonk M J, Dorpmans E, Struik J, Tran Ba Huy P, et al., "Characterization of mucosal biofilms on human adenoid tissues," Laryngoscope, 2008; 118: 128-134.); (Nistico L, Gieseke A, Stoodley P, Hall-Stoodley L, Kerschner JE, and Ehrlich GD, "Fluorescence 'in situ' hybridization for the detection of biofilm in the middle ear and upper respiratory tract mucosa," Methods Mol Biol, 2009; 493: 191-213.).

### Chronic Rhinosinusitis

In Chronic Rhinosinusitis (CRS), mucosal changes with different degrees of denudation in epithelial cells result in a surface favorable for bacterial colonization and biofilm development (Biedlingmaier J, Trifillis A, "Comparison of CT scan and electron microscopic findings on endoscopically harvested middle turbinates," Otolaryngol Head Neck Surg, 1998; 118: 165-173.). Biofilm formation, mainly with Pseudomonas aeruginosa infection, was confirmed in patients who had surgery and continued to have symptoms despite medical treatment (Cryer J, Schipor I, Perloff JR, Palmer JN, "Evidence of bacterial biofilms in human chronic sinusitis," ORL J Otolaryngol Relat Spec, 2004; 66: 155-158.). In patients with CRS having surgery, mucosal biopsies demonstrated different stages of the biofilm by scanning electron microscopy (SEM) in five out of five patients, and all five patients showed aberrant findings of the mucosal surface with various degrees of severity: from disarrayed cilia to complete absence of cilia and goblet cells (Ramadan HH, Sanclement JA, Thomas JG, "Chronic rhinosinusitis and biofilms," Otolaryngol Head Neck Surg, 2005; 132: 414-417.). In most cases of CRS and Pseudomonas aeruginosa biofilms, clinical symptoms were refractory to culture-directed antibiotics, topical steroids, and nasal lavages, and only surgery (mechanical debridement) resulted in significant improvement (Ferguson BJ, Stolz DB, "Demonstration of biofilm in human bacterial chronic rhinosinusitis," Am J Rhinol, 2005; 19: 452-457.).

### Chronic Otitis Media

Chronic Otitis Media (OM) involves inflammation of the middle-ear mucoperiosteal lining and is caused by a variety of microorganisms, including: Streptococcus pneumoniae, Haemophilus influenzae, Moraxella catarrhalis, group A beta-hemolytic streptococci, enteric bacteria, Staphylococcus aureus, Staphylococcus epidermidis, "Pseudomonas aeruginosa, and other organisms; mixed cultures can also be isolated (Feigin RD, Kline MW, Hyatt SR, and Ford III KL, "Otitis media," pp. 174-189. In: Textbook of pediatric infectious diseases, Feigin RD and Cherry JD (ed.), 3-rd ed., vol. 1, 1992, W. B. Saunders Co., Philadelphia, Pa.); (Giebink GS, Juhn SK, Weber ML, and Le CT, "The bacteriology and cytology of chronic otitis media with effusion," Pediatric Infect. Dis., 1982; 1: 98-103.). Chronic OM as a biofilm-related infection was demonstrated in clinical specimens and in animal models. Scanning electron microscopy provided evidence of Haemophilus influenzae biofilm on the middle-ear mucosal surfaces of chinchillas that had been injected with a culture of this organism (Hayes JD, Veeh R, Wang X, Costerton JW, Post JC, and Ehrlich GD, Abstr. 186, Am. Soc. Microbiol. Biofilm, 2000; Conf.2000.); (Hong W, Mason K, Jurcisek J, Novotny L, Bakaletz LO, and Swords WE, "Phosphorylcholine decreases early inflammation and promotes the establishment of stable biofilm communities of nontypeable Haemophilus influenzae strain 86-028NP in a chinchilla model of otitis media," Infect Immun, 2007b; 75: 958-965.). Biofilm aggregates of Streptococcus pneumoniae, Haemophilus influenzae and Moraxella catarrhalis were detected in biopsies of the middle-ear mucosal lining in children with chronic or recurrent OM undergoing TT placement for treatment, but not in the middle-ear mucosal biopsies from patients undergoing surgery for cochlear implantation (Hall-Stoodley L, Hu FZ, Gieseke A, Nistico L, Nguyen D, Hayes J, et al., "Direct detection of bacterial biofilms on the middle-ear mucosa of children with chronic otitis media," JAMA, 2006; 296: 202-211.)

In chronic OM with effusion, the presence of highly viscous fluid in the middle ear requires in many cases the implantation of tympanostomy tubes (TT) to alleviate pressure build-up and hearing loss. Tympanostomy tubes are subject to contamination, and biofilms build up on their inner surfaces. The investigation of colonization and biofilm development by Pseudomonas aeruginosa, Staphylococcus aureus, and Staphylococcus epidermidis on various tympanostomy tubes, provided evidence that all three organisms developed biofilms on the Armstrong silicone and the silver oxide-coated Armstrong-style silicone tubes; Pseudomonas aeruginosa also developed biofilms on the fluoroplastic tubes; only the ionized silicone tubes remained free of contamination and biofilms (Biedlingmaier JF, Samaranayake R, and Whelan P, "Resistance to biofilm formation on otologic implant materials," Otolaryngol Head Neck Surg, 1998; 118: 444-451.). Silver oxide-impregnated silastic tubes lowered the incidence of postoperative otorrhea during the first postoperative week, possibly by preventing adherence and colonization of selected bacteria to the tube, but had no effect on the established infection in the middle ear (Gourin CG and Hubbell RN, "Otorrhea after insertion of silver oxide-impregnated silastic tympanostomy tubes," Arch. Otolaryngol Head Neck Surg, 1999; 125: 446-450.). Bacterial biofilm was also detected on a human cochlear implant (Pawlowski KS, Wawro D, Roland PS, "Bacterial biofilm formation on a human cochlear implant," Otol Neurotol, 2005; 26: 972-975.).

In the lower respiratory tract, microbial biofilms were associated with chronic bronchitis, chronic obstructive pulmonary disease, and pneumonia, especially in patients with cystic fibrosis. Scanning electron microscopy of clinical samples (sputum, bronchiolar lavage, lung and bronchial lining biopsies) demonstrated microbial biofilms either attached to mucosal linings or in the form of bacterial aggregates in mucus covering respiratory epithelium (Lam J, Chan R, Lam K, and Costerton JW, "Production of mucoid microcolonies by Pseudomonas aeruginosa within infected lungs in cystic fibrosis," Infect Immun, 1980; 28: 546-556.); (Martinez-Solano L, Macia MD, Fajardo A, Oliver A, and Martinez JL, "Chronic Pseudomonas aeruginosa infection in chronic obstructive pulmonary disease," Clin Infect Dis, 2008; 47: 1526-1533.); (Starner TD, Zhang N, Kim G, Apicella MA, and McCray PB Jr, "Haemophilus influenzae forms biofilms on airway epithelia: implications in cystic fibrosis," Am J Respir Crit Care Med, 2006; 174: 213-220.); (Worlitzsch D, Tarran R, Ulrich M, Schwab U, Cekici A, Meyer KC, et al., 2002, "Effects of reduced mucus oxygen concentration in airway Pseudomonas infections of cystic fibrosis patients," J Clin Invest, 2002; 109: 317-325.); (Yang L, Haagensen JA, Jelsbak L, Johansen HK, Sternberg C, Høiby N, and Molin S, "In situ growth rates and biofilm development of Pseudomonas aeruginosa populations in chronic lung infections," J Bacteriol, 2008; 190: 2767-2776.).

### Cystic Fibrosis

Cystic fibrosis (CF), a chronic disease of the lower respiratory system, is the most common inherited disease: 70% of patients with CF are defective in the cystic fibrosis transmembrane conductance regulator protein (CFTR), which functions as a chloride ion channel protein, resulting in altered secretions in the secretory epithelia of the respiratory tract. In CF, there is a net deficiency of water, which hinders the upward flow of the mucus layer thus altering mucociliary clearance. Decreased secretion and increased absorption of electrolytes lead to dehydration and thickening of secretions covering the respiratory mucosa (Koch C and Høiby N, "Pathogenesis of cystic fibrosis," Lancet, 1993; 341: 1065-1069.). The hyperviscous mucus is thought to increase the incidence of bacterial lung infections in CF patients. Staphylococcus aureus is usually the first pulmonary isolate from these patients, followed by Haemophilus influenzae. Both of these infections can be treated effectively with antibiotics, but on persistence, they usually form biofilm and predispose the CF-affected lung to colonization with Pseudomonas aeruginosa (colonization rate of∼ 80%) and Burkholderia cepacia with lethal consequences (Govan JR, and Deretic V, "Microbial pathogenesis in cystic fibrosis: mucoid Pseudomonas aeruginosa and Burkholderia cepacia," Microbiol. Rev., 1996; 60: 539-574.). As was demonstrated in clinical studies, both organisms were nonmucoid during initial colonization, but on persistence in the lungs of patients with CF they ultimately undergo changes to mucoid phenotype within a period of time from months to years (Koch C and Høiby N, "Pathogenesis of cystic fibrosis," Lancet, 1993; 341: 1065-1069.). The mucoid material, which was shown to be a polysaccharide substance, later identified as alginate, was transiently produced by laboratory strain of P. aeruginosa, following adherence to the surface (Hoyle BD, Williams LJ, and Costerton JW, "Production of mucoid exopolysaccharide during development of Pseudomonas aeruginosa biofilms," Infect. Immun., 1993; 61: 777-780.). It has been proposed that several in vitro conditions, such as nutrient limitation, the addition of surfactants, and suboptimal levels of antibiotics, may result in mucoidy due to increased production of alginate (May TB, Shinabarger D, Maharaj R, Kato J, Chu L, DeVault JD, Roychoudhury S, Zielinski NA, Berry A, Rothmel RK, Misra TK, and Chakrabarty AM, "Alginate synthesis by Pseudomonas aeruginosa: a key pathogenic factor in chronic pulmonary infections of cystic fibrosis patients," Clin. Microbiol. Rev., 1991; 4: 191-206.). Early antimicrobial treatment with oral ciprofloxacin and inhaled colistin has been shown to postpone chronic infection with Pseudomonas aeruginosa for several years (Koch C and Høiby N, "Pathogenesis of cystic fibrosis," Lancet, 1993; 341: 1065-1069.).

### Periodontal Diseases

Periodontal diseases include infections of the supporting tissues of teeth, ranging from mild and reversible inflammation of the gums (gingiva) to chronic destruction of periodontal tissues (gingiva, periodontal ligament, and alveolar bone) and exfoliation of the teeth. The subgingival crevice (the channel between the tooth root and the gum) is the primary site of periodontal infection and will deepen into a periodontal pocket with the progression of the disease (Lamont RJ and Jenkinson HF, "Life below gum line: pathogenic mechanisms of Porphyromonas gingivalis," Microbiol. Mol. Biol. Rev., 1998; 62: 1244-1263.). Microorganisms isolated from patients with moderate periodontal disease include Fusobacterium nucleatum, Peptostreptococcus micros, Eubacterium timidum, Eubacterium brachy, Lactobacillus spp., Actinomyces naeslundii, Pseudomonas anaerobius, Eubacterium sp. strain D8, Bacteroides intermedius, Fusobacterium spp., Selenomonas sputigena, Eubacterium sp. strain D6, Bacteroides pneumosintes, and Haemophilus aphrophilus, and these bacteria are not found in healthy patients (Moore WEC, Holdeman LV, Cato EP, Smilbert RM, Burmeister JA, and Ranney RR, "Bacteriology of moderate (chronic) periodontitis in mature adult humans," Infect. Immun., 1993; 42: 510-515.). In adult patients with periodontitis, subgingival plaques harbor spirochetes and cocci, and the predominant microorganisms of active lesions in subgingival areas include Fusobacterium nucleatum, Wolinella recta, Bacteroides intermedius, Bacteroides forsythus, and Bacteroides gingivalis (Porphyromonas gingivalis) (Omar AA, Newman HN, and Osborn J, "Darkground microscopy of subgingival plaque from the top to the bottom of the periodontal pocket," J. Clin. Periodontol., 1990; 17: 364-370.); (Dzink JI, Socransky SS, and Haffajee AD, "The predominant cultivable microbiota of active and inactive lesions of destructive periodontal diseases," J. Clin. Periodontol., 1988; 15: 316-323.).

Proteinaceous conditioning films (called acquired pellicle), developed on the exposed surfaces of enamel almost immediately after cleaning of the tooth surface, comprises albumin, lysozyme, glycoproteins, phosphoproteins, lipids, and gingival crevice fluid. Within hours of pellicle formation, single cells of primarily gram-positive cocci and rod-shaped bacteria from the normal oral flora colonize these surfaces, binding directly to the pellicle through the production of extracellular glucans (Kolenbrander PE and London J, "Adhere today, here tomorrow: oral bacterial adherence," J. Bacteriol., 1993; 175: 3247-3252.). After several days, actinomycetes predominate followed by co-aggregation of various microorganisms, resulting in the development of early biofilm with characteristic polysaccharide matrix and polymers of salivary origin, with subsequent (within 2 to 3 weeks) formation of the dental plaque if left undisturbed (Marsh PD, "Dental plaque," pp. 282-300. In: Microbial biofilms. 1995; Lappin-Scott HM and Costerton JW (ed.), Cambridge University Press, Cambridge, United Kingdom.). Plaque can be mineralized with calcium and phosphate ions (called calculus or tartar) and develop more extensively in protected areas (between the teeth, and between the tooth and gum). With the increase of the plaque mass in these protected areas, the beneficial buffering and antimicrobial properties of saliva decrease, leading to dental caries or periodontal disease. Clinical research data show that control of supragingival plaque by professional tooth cleaning and personal hygienic efforts can prevent gingival inflammation and adult periodontitis (Corbet EF and Davies WIR, "The role of supragingival plaque in the control of progressive periodontal disease," J. Clin. Periodontol., 1993; 20: 307-313.).

### Chronic Bacterial Prostatitis

The prostate gland may become infected by bacteria ascended from the urethra or by reflux of infected urine into the prostatic ducts emptying into the posterior urethra (Domingue GJ and Hellstrom WJG, "Prostatitis," Clin. Microbiol. Rev., 1998; 11: 604-613.). If bacteria were not eradicated with antibiotic therapy at the early stage of infection, they continue to persist and can form sporadic microcolonies and biofilms that adhere to the epithelial cells of the prostatic duct system, resulting in chronic bacterial prostatitis. The microorganisms involved in this process include: E. coli (most common isolate), Klebsiella, Enterobacteria, Proteus, Serratia, Pseudomonas aeruginosa, Enterococcus fecalis, Bacteroides spp., Gardnerella spp., Corynebacterium spp., and Coagulase-negative Staphylococci (CoNS) (Nickel JC, Costerton JW, McLean RJC, and Olson M, "Bacterial biofilms: influence on the pathogenesis, diagnosis, and treatment of the urinary tract infections," J. Antimicrob. Chemother., 1994; 33 (Suppl. A): 31-41.). The biopsies from patients with chronic bacterial prostatitis examined by either scanning electron microscopy or transmission electron microscopy, demonstrated bacteria present in glycocalyx-encasted microcolonies, firmly adherent to the ductal and acinar mucosal layers (Nickel JC and Costerton JW, "Bacterial localization in antibiotic-refractory chronic bacterial prostatitis," Prostate, 1993; 23: 107-114.). Sporadic microcolonies of CoNS in the intraductal space have been shown to be enveloped in a dehydrated slime matrix (Nickel JC and (Costerton JW, "Coagulase-negative staphylococcus in chronic prostatitis," J. Urol., 1992; 147: 398-401.). Treatment failures are common in chronic bacterial prostatitis due to the local environment and biofilm formation, with changes in bacterial metabolism and possible development of resistance to antimicrobials. In order to increase the effectiveness of the antimicrobial treatment, it has been proposed to deliver higher antibiotic concentrations directly to the biofilm within the prostatic ducts (Nickel JC, Costerton JW, Mclean RJC, and Olson M, "Bacterial biofilms: influence on the pathogenesis, diagnosis, and treatment of the urinary tract infections," J. Antimicrob. Chemother., 1994; 33 (Suppl. A): 31-41.).

### Biofilms on Medical Devices

Over the last 20 years, biofilms on various medical devices, including prosthetic heart valves, central venous catheters, urinary (Foley) catheters, contact lenses, intrauterine devices, and dental unit water lines, have been studied using viable bacterial culture techniques and scanning electron microscopy, and for certain devices (contact lenses and urinary catheters) additional evaluation of susceptibility of various materials to bacterial adhesion and biofilm formation have also been implemented (Costerton JW, Stewart PS, and Greenberg EP, "Bacterial biofilms: a common cause of persistent infections," Science, 1999; 284: 1318-1322.); (Donlan RM and Costerton JW, "Review. Biofilms: Survival mechanisms of clinically relevant microorganisms," Clinical Microbiology Reviews, Apr. 2002; 167-193.).

### Prosthetic Heart Valves

Prosthetic valve endocarditis (PVE) is a microbial infection of the valve and surrounding tissues of the heart, ranging between 0.5% and 4%, and is similar for both types of valves currently used - mechanical valves and bioprostheses (Douglas JL and Cobbs CG, "Prosthetic valve endocarditis," pp.375-396. In: Infective endocarditis, Kaye D. (ed.), 2-nd ed., 1992; Raven Press LTD., New York, N.Y.). Tissue damage resulting from surgical implantation of the prosthetic valve, leads to accumulation of platelets and fibrin at the suture site and on the device, providing a favorable environment for bacterial colonization and biofilm development. PVE is predominantly caused by microbial colonization of the sewing cuff fabric. The microorganisms commonly invade the valve annulus, potentially promoting separation between the valve and the tissue resulting in leakage. Infectious microorganisms involved in PVE include Staphylococcus epidermidis (at the early stages), followed by Streptococci, CoNS, Enterococci, Staphylococcus aureus, gram-negative Coccobacilli, fungi, and Streptococcus viridans spp. (the most common microorganism isolated during late PVE) (Hancock EW, "Artificial valve disease," pp. 1539-1545. In: The heart arteries and veins; Schlant RC, Alexander RW, O'Rourke RA, Roberts R, and Sonnenblick EH (ed.), 8-th ed., 1994; vol.2. McGraw-Hill, Inc., New York, N.Y.); (Illingworth BL, Twenden K, Schroeder RF, and Cameron JD, "In vivo efficacy of silver-coated (silzone) infection-resistant polyester fabric against a biofilm producing bacteria, Staphylococcus epidermidis, J. Heart Valve Dis., 1998; 7: 524-530.); (Karchmer AW and Gibbons GW, "Infections of prosthetic heart valves and vascular grafts," pp.213-249. In: Infections associated with indwelling medical devices; Bisno AL and Waldovogel FA (ed.), 1994, 2-nd ed. American Society for Microbiology, Washington, D.C.).

### Central Venous Catheters

For Central Venous Catheters (CVCs), the device-related infection rate is 3% to 5%. Infectious biofilms are universally present on CVCs and can be associated with either the outside surface of the catheter or the inner lumen. Colonization and biofilm formation may occur within 3 days of catheterization. Short-term catheters (in place for less than 10 days) usually have more extensive biofilm formation on the external surfaces, and long-term catheters (up to 30 days) have more extensive biofilm on the internal lumen. (Raad II, Costerton JW, Sabharwal, Sacilowski UM, Anaissie W, and Bodey GP, "Ultrastructural analysis of indwelling vascular catheters: a quantitative relationship between luminal colonization and duration of placement," J. Infect. Dis., 1993; 168: 400-407.). Colonizing microorganisms originate either from the skin insertion site, migrating along the external surface of the device, or from the hub, due to manipulation by health care workers, migrating along the inner lumen (Elliott TSJ, Moss HA, Tebbs SE, Wilson IC, Bonser RS, Graham TR, Burke LP, and Faroqui MH, "Novel approach to investigate a source of microbial contamination of central venous catheters," Eur. J. Clin. Microbiol. Infect. Dis., 1997; 16: 210-213.). Because the device is in direct contact with the bloodstream, the surface becomes coated with platelets, plasma and tissue proteins such as albumin, fibrinogen, fibronectin, and laminin, forming conditioning films to which the bacteria are adherent: Staphylococcus aureus adheres to fibronectin, fibrinogen, and laminin, and Staphylococcus epidermidis, adheres only to fibronectin. Organisms colonizing CVCs include CoNS, Staphylococcus aureus, Pseudomonas aeruginosa, Klebsiella pneumoniae, Enterococcus fecalis, and Candida albicans (Elliott TSJ, Moss HA, Tebbs SE, Wilson IC, Bonser RS, Graham TR, Burke LP, and Faroqui MH, "Novel approach to investigate a source of microbial contamination of central venous catheters," Eur. J. Clin. Microbiol. Infect. Dis., 1997; 16: 210-213.).

### Urinary Catheters

Urinary catheters are subject to bacterial contamination regardless of the types of the catheter systems. In open systems, the catheter draining into an open collection container becomes contaminated quickly, and patients commonly develop Urinary Tract Infection (UTI) within 3 to 4 days. In closed systems, when the catheter empties in a securely fastened plastic collecting bag, the urine from the patient can remain sterile for 10 to 14 days in approximately half the patients (Kaye D and Hessen T, "Infections associated with foreign bodies in the urinary tract," pp.291-307. In: Infections associated with indwelling medical devices; Bisno AL and Waldovogel FA (ed.), 1994; 2-nd ed., American Society for Microbiology, Washington, D. C.). Regardless of the type of the system, with short-term catheterization (up to 7 days), 10% to 50% of patients develop UTI, and with long-term catheterization (28 days and longer) essentially all patients develop UTI (Stickler DJ, "Bacterial biofilms and the encrustation of urethral catheters," Biofouling, 1996; 94: 293-305.). The risk of catheter-associated UTI increases by approximately 10% for each day the catheter is in place. Initially, catheters are colonized by a single microorganism, such as Staphylococcus epidermidis, Enterococcus fecalis, E. coli, Proteus mirabilis. Later, the number and diversity of bacteria increase, with mixed communities containing Providencia stuartii, Pseudomonas aeruginosa, Proteus mirabilis, Klebsiella pneumoniae, Morganella morganii, Acinetobacter calcoaceticus, and Enterobacter aerogenes (McLean RJC, Nickel JC, and Olson ME, "Biofilm associated urinary tract infections," pp. 261-273. In: Microbial biofilms; 1995, Lappin-Scott HM and Costerton JW (ed.), Cambridge University Press, Cambridge, United Kingdom.).

Both in vivo and in vitro studies by scanning electron microscopy and transmission electron microscopy provide evidence for biofilm formation on catheters. The thickness of biofilm on silicone and silicone-coated Foley catheters from patients undergoing long-term catheterization ranges from 200µm to 500µm, with the thickest biofilms formed by E. coli and Klebsiella pneumoniae (up to 490µm). The thinnest biofilms were formed by Morganella morganii and diphtheroids (the average ∼ 10µm), and these biofilms were also patchy (Ganderton L, Chawla J, Winters C, Wimpenny J, and Stickler D, "Scanning electron microscopy of bacterial biofilms on indwelling bladder catheters," Eur. J. Clin. Microbiol. Infect. Dis., 1992; 11: 789-796.).

Urinary catheter biofilms are unique, because certain microorganisms produce enzyme urease which hydrolyzes the urea of the urine to form free ammonia, thus raising the local pH and allowing precipitation of minerals hydxoxyapatite (calcium phosphate) and struvite (magnesium ammonium phosphate). These minerals become deposited in the catheter biofilms, forming a mineral encrustation which can completely block a urinary catheter within 3 to 5 days (Tunney MM, Jones DS, and Gorman SP, "Biofilm and biofilm-related encrustations of urinary tract devices," Methods Enzymol., 1999; 310: 558-566.). The primary urease-producing organisms in urinary catheters are Proteus mirabilis, Morganella morganii, Pseudomonas aeruginosa, Klebsiella pneumoniae, and Proteus vulgaris. Mineral encrustations were observed only in catheters containing these bacteria Stickler D, Morris N, Moreno MC, and Sabbuba N, "Studies on the formation of crystalline bacterial biofilms on urethral catheters," Eur. J. Clin. Microbiol. Infect. Dis., 1998; 17: 649-652.); (Stickler D, Ganderton L, King J, Nettleton J, and Winters C, "Proteus mirabilis biofilms and the encrustation of urethral catheters," Urol. Res., 1993; 21: 407-411.).

### Contact Lenses

Bacteria adhere readily to both types of contact lenses: soft contact lenses (made of either hydrogel or silicone) and hard contact lenses constructed of polymethylmethacrylate. Initial adhesion of Pseudomonas aeruginosa to hydrogel contact lenses, resulted within 2 hours in biofilm formation with characteristic extracellular matrix polymers observed by transmission electron microscopy and ruthenium red staining (Miller MJ and Ahearn G, "Adherence of Pseudomonas aeruginosa to hydrophilic contact lenses and other substrata," J. Clin. Microbiol., 1987; 25: 1392-1397.). The degree of attachment depended on various factors, including the nature of the substrate, pH, electrolyte concentration, ionic charge of the polymer, and bacterial strain tested.

Organisms that have been shown to adhere to contact lenses include: Pseudomonas aeruginosa, Staphylococcus aureus, Staphylococcus epidermidis, Serratia spp., E. coli, Proteus spp., and Candida spp. (Dart JKG, "Contact lens and prosthesis infections," pp. 1-30. In: Duane's foundations of clinical ophthalmology; Tasman W and Jaeger EA (ed.), 1996; Lippincott-Raven, Philadelphia, PA.). An established biofilm was detected on the lens removed from a patient with P. aeruginosa keratitis, as well as from the patients with clinical diagnosis of microbial keratitis, in several cases containing multiple species of bacteria or bacteria and fungi (Stapleton F and Dart J, "Pseudomonas keratitis associated with biofilm formation on a disposable soft contact lens," Br. J. Ophthalmol., 1995; 79: 864-865.); (McLaughlin-Borlace L, Stapleton F, Matheson M, and Dart JKG, "Bacterial biofilm on contact lenses and lens storage cases in wearers with microbial keratitis," J. Appl. Microbiol., 1998; 84: 827-838.).

The lens case has been implicated as the primary source of microorganisms for contaminated lenses and lens disinfectant solutions, with contaminated storage cases in 80% of asymptomatic lens users (McLaughlin-Borlace L, Stapleton F, Matheson M, and Dart JKG, "Bacterial biofilm on contact lenses and lens storage cases in wearers with microbial keratitis," J. Appl. Microbiol., 1998; 84: 827-838.). Also, the identical organisms were isolated from the lens cases and the corneas of infected patients. Additionally, protozoan Acanthamoeba has been shown to be a component of these biofilms (Dart JKG, "Contact lens and prosthesis infections," pp. 1-30. In: Duane's foundations of clinical ophthalmology; Tasman W and Jaeger EA (ed.), 1996; Lippincott-Raven, Philadelphia, PA.); (McLaughlin-Borlace L, Stapleton F, Matheson M, and Dart JKG, "Bacterial biofilm on contact lenses and lens storage cases in wearers with microbial keratitis," J. Appl. Microbiol., 1998; 84: 827-838.).

### Dental Unit Water Lines

Dental procedures may expose both patients and dental professionals to opportunistic and pathogenic organisms originating from various components of the dental unit. Small-bore flexible plastic tubing supplies water (municipal or from separate reservoirs containing distilled, filtered, or sterile water) to different hand pieces (air-water syringe, the ultrasonic scaler, the high-speed hand piece), and elevated bacterial counts were detected in all these systems (Barbeau J, Tanguay R, Faucher E, Avezard C, Trudel L, Cote L, and Prevost AP, "Multiparametric analysis of waterline contamination in dental units," Appl. Environ. Microbiol., 1996; 62: 3954-3959.); (Furuhashi M and Miyamae T, "Prevention of bacterial contamination of water in dental units," J. Hosp. Infect., 1985; 6: 81-88.); (Mayo JA, Oertling KM, and Andrieu SC, "Bacterial biofilm: a source of contamination in dental air-water syringes," Clin. Prev. Dent., 1990; 12: 13-20.); (Williams HN, Kelley J, Folineo D, Williams GC, Hawley CL, and Sibiski J, "Assessing microbial contamination in clean water dental units and compliance with disinfection protocol," JADA, 1994; 125: 1205-1211.).

Organisms generally isolated from dental water units include Pseudomonas spp., Flavobacterium spp., Acinetobacter spp., Moraxella spp., Achromobacter spp., Methylobacterium spp., Rhodotorula spp., hyphomycetes (Cladosporium spp., Aspergillus spp., and Penicillium spp.), Bacillus spp., Streptococcus spp., CoNS, Micrococcus spp., Corynebacterium spp., and Legionella pneumophila (Tall BD, Williams HN, George KS, Gray RT, and Walch M, "Bacterial succession within a biofilm in water supply lines of dental air-water syringes," Can. J. Microbiol., 1995; 41: 647-654.); (Whitehouse RLS, Peters E, Lizotte J, and Lilge C, "Influence of biofilms on microbial contamination in dental unit water," J. Dent., 1991; 19: 290-295.); (Mills SEP, Lauderdale W, and Mayhew RB, "Reduction of microbial contamination in dental units with povidone-iodine 10%," JADA, 1986; 113: 280-284.); (Atlas RM, Williams JF, and Huntington MK, "Legionella contamination of dental-unit waters," Appl. Environ. Microbiol., 1995; 61: 1208-1213.); (Callacombe SJ and Fernandes LL, "Detecting Legionella pneumophila in water systems: a comparison of various dental units," JADA, 1995; 126: 603-608.); (Pankhurst CL, Philpott-Howard JN, Hewitt JH, and Casewell MW, "The efficacy of chlorination and filtration in the control and eradication of Legionella from dental chair water systems," J. Hosp. Infect., 1990; 16: 9-18.). The variety of microorganisms observed, were embedded in an apparent polysaccharide matrix (Whitehouse RLS, Peters E, Lizotte J, and Lilge C, "Influence of biofilms on microbial contamination in dental unit water," J. Dent., 1991; 19: 290-295.). Also, amebic trophozoites and cysts, and nematodes (in one biofilm sample) were also observed (Santiago JI, Huntington MK, Johnston AM, Quinn RS, and Williams JF, "Microbial contamination of dental unit waterlines: short- and long-term effects of flushing," Gen. Dent., 1994; 42: 528-535.). A positive correlation was found between biofilm and water counts, and by 180 days of exposure, a thick, multiple layer of extracellular polymeric substances covered the entire surface of the dental unit water line (Tall BD, Williams HN, George KS, Gray RT, and Walch M, "Bacterial succession within a biofilm in water supply lines of dental air-water syringes," Can. J. Microbiol., 1995; 41: 647-654.). Biofilms containing extensive extracellular polymer matrix and both mixed skin flora and aquatic bacteria, were also detected on the inner lumen of saliva ejectors (Barbeau J, ten Bocum L, Gauthier C, and Prevost AP, "Cross contamination potential of saliva ejectors used in dentistry," J. Hosp. Infect., 1998; 40: 303-311.).

### Methods of Treating Biofilms and Biofilm-Based Infections

Many biofilm control strategies have been proposed, applied mostly to biofilm formed on various medical devices, including long term antibiotics for patients using these devices, various antimicrobials to cover the surfaces of devices, various polymer materials, ultrasound, and low-strength electrical fields along with disinfectants.

For biofilm-based infections in the human body, a few approaches aimed to either eradicate or penetrate the extracellular polymeric substances have been offered: for example, a mixture of enzymes was effective in eradicating laboratory-grown biofilms of several different organisms (Johansen CP, Falholt P, and Gram L, "Enzymatic removal and disinfection of bacterial biofilm," Appl. Environ. Microbiol., 1997; 63: 3724- 3728.). Another more precise approach was identifying the polysaccharides for a specific organism in the biofilm and treating the biofilm with that enzyme: for example, the specific enzyme alginate lyase allowed more effective diffusion of gentamycin and tobramycin through alginate, the biofilm polysaccharide of mucoid Pseudomonas aeruginosa (Hatch RA, and Schiller NL, "Alginate lyase promotes diffusion of aminoglycosides through the extracellular polysaccharide of mucoid Pseudomonas aeruginosa," Antimicrob. Agents Chemother., 1998; 42: 974-977.). In addition, for the management of biofilm infections, various antibiotics have been examined extensively in vitro and in vivo, including aminoglycosides, fluoroquinolones, macrolides, as well as the latest protein synthesis inhibitors (Linezolid and Quinupristin) clinically available and appear promising for treatment of in vivo biofilm infections (In: Biofilms, Infection, and Antimicrobial Therapy; Edited by Pace JL, Rupp ME, and Finch RG; Boca Raton, FL: CRC Press, 2006. Chapter 18, page 360.).

A review of recent patent literature summarizes citations under six categories of current treatment approaches: 1) antibiotics and small molecule inhibitors of new and established biofilms, 2) quorum sensing and signaling molecules inhibitors, 3) surface coating substances for inhibition of biofilm formation, 4) antibodies and vaccines for infectious biofilm treatment, 5) enzymes for degrading biofilms, and 6) bacteriophage treatment of infectious biofilms (Lynch AS and Abbanat D, "New antibiotic agents and approaches to treat biofilm-associated infections," Expert Opin. Ther. Patents, 2010; 20(10): 1373-1387.).

Additional approaches involve the use of various natural substances and combined technologies. For example, naturally occurring impediments to biofilm adhesion have been proposed such as, oral-ficin, a cysteine protease derived from the Ficus glabrata tree, which prevents biofilm-forming bacteria from adhering to surfaces (Potera C, "A Potpourri of Probing and Treating Biofilms of the Oral Cavity," Microbe Magazine, Oct. 2009.). The ability of honey to prevent quorum sensing and thereby interfere with the formation or maintenance of biofilms suggests it can be a candidate substance for the management of infected wounds ("The role of biofilm in wounds," a thesis submitted to the University of Wales, Cardiff, UK, in candidature for Ph.D. by Okhiria OA, May 2010, Chapter 5: Antimicrobial effect of honey on biofilm and quorum sensing: 190-234.).

An example of the use of combined technologies is the treatment of biofilm infections on implants using ultrasound in concert with antibiotics (Carmen JC, Roeder BL, Nelson JL, Robison Ogilvie RL, Robison RA, Schaalje GB, and Pitt WG, "Treatment of Biofilm Infections on Implants with Low-frequency Ultrasound and Antibiotics," Am J Infect Control. 2005, March; 33(2): 78-82.).

### Methods of Addressing Biofilm Contamination of Medical Equipment

Bacterial and fungal biofilms develop on the various types of medical equipment. This includes medical diagnostic devices, such as: stethoscopes, colposcopes, nasopharyngoscopes, angiography catheters, endoscopes, angioplasty balloon catheters; and various permanent, semi-permanent, and temporary indwelling devices, such as: contact lenses, intrauterine devices, dental implants, urinary tract prostheses and catheters, peritoneal dialysis catheters, indwelling catheters for hemodialysis and for chronic administration of chemotherapeutic agents (Hickman catheters), cardiac implants (pacemakers, prosthetic heart valves, ventricular assisting devices - VAD), synthetic vascular grafts and stents, prostheses, internal fixation devices, percutaneous sutures, tracheal and ventilator tubing, dispensing devices such as nebulizers, and cleaning devices such as sterilizers. Summarized herein are the current methods employed to diminish the presence of microbial biofilms and associated pathogens on medical equipment.

### Implants

Biofilm infections associated with indwelling medical devices and implants are difficult to resolve using conventional antibiotics. Antibiotic treatment requires lengthy periods of administration, with combined antibiotics at high dose, or the temporary surgical removal of the device or associated tissue. Newer developments, aimed at interfering with the colonization process comprise, for example, new biomaterials, the co-application of acoustic energy or low-voltage electric currents with antibiotics and the development of specific anti-biofilm agents (Jass J, Surman S, and Walker JT, "Medical biofilms: detection, prevention, and control," Vol. 2., John Wiley, 2003: 261.).

### Central Venous Catheters

Several studies have examined the effect of various types of antimicrobial treatment in controlling biofilm formation on venous catheters. The methods and materials used include adding disinfectant to physiological flush of catheters for elimination of microbial colonization (Freeman R, Gould FK. "Infection and intravascular catheters," [letter]. J. Antimicrob. Chemother., 1985; 15: 258.), impregnation of catheters with polyantimicrobials (Darouiche RO et al. , "A comparison of two antimicrobial-impregnated central venous catheters," N Engl J Med, 1999; 340: 1-8.), coating of catheters with surfactants to bond antibiotics to catheter surfaces (Kamal G.D., Pfaller M.A., Rempe L.E., Jebson P.J.R., "Reduced intravascular catheter infection by antibiotic bonding. A prospective, randomized, controlled trial", JAMA, 1991; 265: 2364-2368.), and the use of an attachable subcutaneous cuff containing silver ions inserted after local application of polyantibiotic (Flowers R.H., Schwenzer K.J., Kopel R.F., Fisch M.J., Tucker S.I., Farr B.M., "Efficacy of an attachable subcutaneous cuff for the prevention of intravascular catheter-related infection", JAMA, 1989; 261: 878-883.).

### Prosthetic Heart Valves

The pathogenesis of infection associated with implanted heart valves is related to the interface between the valve and surrounding tissue. Specifically, because implantation of a mechanical heart valve causes tissue damage at the site of its installation, microorganisms have an increased tendency to colonize such locations (Donlan RM, "Biofilms and Device-Associated Infections," Emerging Infectious Diseases Journal, Mar-Apr 2001; Vol. 7, No. 2: 277-281.). Hence, biofilms resulting from such infections tend to favor development on the tissue surrounding the implant or the sewing cuff fabric used to attach the device to the tissue. Silver coating of the sewing cuff has been found to reduce such infections (Illingworth BL, Tweden K, Schroeder RF, Cameron JD, "In vivo efficacy of silver-coated (Silzone) infection-resistant polyester fabric against a biofilm-producing bacteria, Staphylococcus epidermidis", J Heart Valve Dis 1998; 7: 524. Abstract); (Carrel T, Nguyen T, Kipfer B, Althaus U, "Definitive cure of recurrent prosthetic endocarditis using silver-coated St. Jude medical heart valves: a preliminary case report," J Heart Valve Dis., 1998; 7: 531. Abstract.).

### Urinary Catheters

Conventional approaches to the treatment of urinary catheter biofilms include: the use of antimicrobial ointments and lubricants, instillation or irrigation of the bladder with antimicrobials, use of the collection bags containing antimicrobial agents, catheter impregnation with antimicrobial agents, and the use of systemic antibiotics (Kaye D, Hessen MT, "Infections associated with foreign bodies in the urinary tract," In: Bisno A.L., Waldovogel F.A., editors. Infections associated with indwelling medical devices. 2nd ed. Washington: American Society for Microbiology; 1994; pp. 291-307.). Such approaches have been found to have limited efficacy, although silver impregnation of catheters has been found to delay onset of bacteriuria (Donlan RM, "Biofilms and Device-Associated Infections," Emerging Infectious Diseases Journal, Mar-Apr 2001; Vol. 7, No. 2: 277-281.). From various materials used for catheter construction, silicone catheters obstruct less often than latex, Teflon, or silicone-coated latex in patients prone to catheter encrustation (Sedor J and Mulholland SG, "Hospital-acquired urinary tract infections associated with indwelling catheter," Urol. Clin. N. Am., 1999; 26: 821-828.).

A new product, the UroShieldTM System, produced by NanoVibronix uses low cost disposable ultrasonic actuators which energize all surfaces of the catheter thereby interfering with the attachment of bacteria, the initial step in biofilm formation (Nagy K, Köves B, Jäckel M, Tenke P, "The effectiveness of acoustic energy induced by UroShield device in the prevention of bacteriuria and the reduction of patient's complaints related to long-term indwelling urinary catheters," Poster presentation at 26th Annual Congress of the European Association of Urology (EAU); Vienna, March 2011: No. 483. Abstract.).

### Dialysis systems

The development of biofilms throughout hemodialysis systems has been substantiated. In fact, some cases have been suspicious for the outbreak of infection within dialysis centers. Furthermore, the endotoxins and other cytokines in these biofilms can cross the dialysis membrane and trigger the inflammatory response in the patients (Vincent FC, Tibi AR, and Darbord JC. "A bacterial biofilm in a hemodialysis system. Assessment of disinfection and crossing of endotoxins," ASAIO Trans., 1989; 35: 310-313.). In a study specific to the removal of biofilms from dialysis tubing, the efficacy of 21 different decontamination procedures was ascertained with the most effective treatment determined to be an acid pre-treatment, followed by use of a concentrated bleach solution; treatments performed at high temperature did not improve the removal of biofilm (Marion-Ferey K, et al., "Biofilm removal from silicone tubing: an assessment of the efficacy of dialysis machine decontamination procedures using an in vitro model," Journal of Hospital Infection, 2003; 53(1): 64-71.).

Given the challenge of removing biofilms from the in-place water systems found in clinical environments, a multi-step cleaning (removal of organic material), descaling (removal of inorganic material), and disinfection (removal of microorganisms) process is suggested. The most common current protocols include the following: a) citric acid followed by bleach, b) bleach alone, c) peracetic acid with acetic acid and hydrogen peroxide (PAA), d) citric acid followed by autoclaving, e) citric acid at elevated temperature, f) glycolic acid at elevated temperature, g) hot water, and h) citric acid followed by PAA. All of these disinfection protocols appear to be highly efficient with respect to microbial killing, but were inefficient in reducing the amount of biofilm on affected surfaces.

No treatment thus far has shown complete biofilm removal (and consequently endotoxins) from silicone surfaces. Descaling by itself is inadequate, even at high temperature. Bleach appears to be a relatively good solitary agent for biofilm removal. Additionally, UV irradiation has been shown to have limited impact on biofilms; and ozone has demonstrated a higher removal efficacy, but limited biofilm killing. It has been postulated that destruction of both the bacteria and associated endotoxins may be possible if super-oxidative concentrations can be achieved ("The Role of Biofilms in Device-Related Infections," Ed. By Shirtliff M and Leid JG; Springer-Verlag, Berlin, 2009.).

### Endoscopes

In a comparative study of the efficiency of numerous detergents to remove endoscope biofilms, it was determined that "many commonly used enzymatic cleaners fail to reduce the viable bacterial load or remove the bacterial EPS" (Vickery K, Pajkos A, and Cossart Y," Removal of biofilm from endoscopes: evaluation of detergent efficiency, "Am J Infect Control. 2004, May; 32(3): 170-176.). Only one cleaner containing no enzymes (produced by Whiteley Medical, Sydney, Australia) significantly reduced bacterial viability and residual bacterial exopolysaccharide matrix.

Noteworthy is U.S. Patent 6,855,678, in which it is disclosed that through the use of scanning electron microscopy, it has been observed that biofilm consists of a number of layers and most importantly, there exists a thin layer of biofilm which is adjacent and attaches tightly to the surface of medical apparatus. The treatment formulation advocated herein includes in combination surfactants, solvents, co-solvents, nitrogen containing biocide, and organic chelating agents. This composition provides a simple non-corrosive, near neutral chemical detergent product that reliably cleans and disinfects endoscopes and other-medical apparatus. The hypothesized method of action is that a) the solvent and co-solvent (example solvents include low molecular weight polar water soluble solvents such as primary and secondary alcohols, glycols, esters, ketones, aromatic alcohols, and cyclic nitrogen solvents containing 8 or less carbon atoms, example co-solvents include low molecular weight amine, amide, and methyl and ethyl derivatives of amides) act to swell the biofilm, b) the organic chelating agent in combination with the surfactant increases the ability of the nitrogen containing biocide to penetrate the biofilm, and c) the organic chelating agent in combination with the nitrogen containing biocide act to work synergistically to dislodge the biofilm and/or kill the microorganisms therein.

### Contact Lenses

Various cleaning solutions were tested against bacterial biofilms on contact lens storage cases, including quaternary ammonium compounds, chlorhexidine gluconate, and hydrogen peroxide 3%. Hydrogen peroxide 3% was most effective in inactivating 24 hr-old biofilms formed by Pseudomonas aeruginosa, Staphylococcus epidermidis, and Streptococcus pyogenes. Biofilm of Candida albicans was highly resistant to all of these treatments, and Serratia marcescens could grow in chlorhexidine disinfectant solutions (Wilson LA., Sawant AD, and Ahearn DG, "Comparative efficacies of soft contact lens disinfectant solutions against microbial films in lens cases," Arch. Ophthalmol., 1991; 109: 1155-1157.); (Gandhi PA, Sawant AD, Wilson LA, and Ahearn DG, "Adaptation and growth of Serratia marcescens in contact lens disinfectant solutions containing chlorhexidine gluconate," Appl. Environ. Microbiol. , 1993; 59: 183-188.). It has been found that sodium salicylate decreased initial bacterial adherence to lenses and lens cases (Farber BF, His-Chia H, Donnenfield ED, Perry HD, Epstein A, and Wolff A, "A novel antibiofilm technology for contact lens solutions," Ophthalmology, 1995; 102: 831-836.).

### Dental Unit Water Lines

Dental unit water lines are ideal for colonization with aquatic bacteria and biofilm formation due to their small diameter, very high surface-to-volume ratio, and relatively low flow rates. Currently used flushing as treatment for reducing planktonic bacterial load that originates from the tubing biofilm, does not provide sufficient results, and flushing alone is ineffective (Santiago JI, Huntington MK, Johnston AM, Quinn RS, and Williams JF, "Microbial contamination of dental unit waterlines: short-and long-term effects of flushing," Gen. Dent., 1994; 42: 528-535.). Added povidone-iodine reduced contamination between 4 and 5 log fewer bacteria per ml initially, but the levels returned to pretreatment within 22 days (Mills SE, Lauderdale PW, and Mayhew RB, "Reduction of microbial contamination in dental units with povidone-iodine 10%," JADA, 1986; 113: 280-284.). Treatment with 0.5 to 1 ppm free chlorine for 10 min. each day reduced normal bacterial counts by 2 logs from pretreatment levels, but the counts increased again after chlorination was discontinued (Feigin RD and Henriksen K, "Methods of disinfection of the water system of dental units by water chlorination," J. Dent. Res., 1988; 67: 1499-1504.). Chlorination with bleach (1:10 solution) of water systems already contaminated with bacterial biofilms was ineffective in removing them (Murdoch-Kinch CA, Andrews NA, Atwan S, Jude R, Gleason MJ, and Molinari JA, "Comparison of dental water quality management procedures," JADA, 1997; 128: 1235-1243.).

### Biofilms in Industrial Applications (Pipelines, Marine Biofouling, Food Sanitation, and HVAC)

Industrial systems suffer a number of deleterious effects due to the presence of biofilms. For heating and cooling systems, as well as oil, water, and gas distributions systems, these effects include flow restrictions in pipelines, flow contamination, and corrosion. For marine systems such as ships, biofouling of hulls can lead to tremendous loss of ship fuel efficiency owing to increased drag of the hull.

### Current Approaches for Treating Biofilms in Water, Oil and Gas Distribution Systems

In industrial systems for the distribution of water, oil, and gas, biofilms can form heavy biomass that can reduce the effective diameter of a pipe or other conduit at a particular point or increase friction along the flow path in the conduit. This increases resistance to flow through the conduit, reduces the flow volume, increases pump power consumption, decreasing the efficiency of industrial operations. Further, this biomass can serve as a source of contamination to flowing water or oil. Additionally, most biofilms are heterogeneous in composition and structure which leads to the formation of cathodic and anodic sites within the underlying conduit metal thereby contributing to corrosion processes.

Currently, for pipeline treatment of biofilms, there is a trend to use strong oxidizing biocides such as chlorine dioxide in cooling systems and ozone in water distribution systems since low levels of chlorine have been found to be ineffective against biofilms. Also, a number of non-oxidizing biocides are available, which are effective but their long-term effects on the environment are still unclear. New techniques for biofilm control, such as ultrasound, electrical fields, hydrolysis of EPS and methods altering biofilm adhesion and cohesion are still in their infancy at the laboratory level and are yet to be successfully demonstrated in large industrial systems (Sriyutha Murthy P and Venkatesan R, "Industrial Biofilms and Their Control". In: Marine and Industrial Biofouling; Editors: Fleming H, Murphy P, Venkatesan R, and Cooksey K; Springer-Verlag, 2010.).

One of the major economic losses faced by the oil and gas companies is due to pipeline corrosion. The internal corrosion of the pipelines is basically caused by sulfate reducing bacteria (SRB). SRB are anaerobic and responsible for most instances of accelerated corrosion damage. For biofilms created by SRB, some newer strategies include the use of: a) calcium or sodium nitrates which encourage more benign nitrate reducing bacteria to compete with SRB, b) molybdate as a metabolic inhibitor preventing sulfate reduction, c) anthraquinone which prohibits sulfide production and its incorporation into the biofilm, and d) dispersants such as filming amine technology which prevent biofilm adhesion. Also, since there is no continuous water phase in oil pipelines (under typical flow conditions) by which to dose bactericides, the use of water-oil emulsions have been suggested ("Petroleum Microbiology"; edited by Ollivier B and Magot M, ASM Press, 2005.).

An example of the more recent biofilm altered adhesion concepts includes the disclosure of International Patent Application PCT/US2006/028353 describing a nontoxic, peptide-based biofilm inhibitor that prevents Pseudomonas aeruginosa colonization of stainless steel (and likely a wide variety of other metal surfaces) and non-metalic surfaces. The compositions and methods describe a very high affinity peptide ligand that binds specifically to stainless steel and other surfaces to prevent Pseudomonas biofilm formation. Another example of an inhibitor of biofilm adhesion is the technology being developed by Australian firm BioSignal Ltd. involving the use of furanones from the red seaweed Delisea pulchra, which effectively avoids a broad spectrum of bacterial infections without inciting any bacterial resistance to its defensive chemistry. Furanones produced by this seaweed, bind readily to the same specific protein-covered bacterial receptor sites that receive the bacterial signaling molecules (N-acyl homoserine lactone) which normally induce surface colonization. BioSignal Ltd. is targeting the use of synthetic furanones to block bacterial communication and thereby prevent bacteria from forming groups and biofilms in applications including pipelines, HVAC, and water lines treatment.

### Methods of Decontamination of Food Processing, Storage, and Transport Systems in the Food Iindustry

In addition to the more conventional means of decontamination discussed above for other industrial applications, recently, the food industry has embarked upon the use of enzyme-based schemes that have been carried over from the bio-processing of food stuffs. Specifically, efforts have been undertaken to find ways to enzymatically degrade the EPS itself and thereby contribute to the removal of biofilms. Largely, these efforts have been directed at destruction of the polysaccharide framework of the EPS. A premier example is found in the U. S. Patent Application 20110104141 to Novozyme which discloses the use of alpha-amylase as a primary enzyme for the breakdown of biofilm polysaccharides with the potential inclusion of additional enzymes such as aminopeptidase, amylase, carbohydrase, carboxypeptidase, catalase, cellulase, chitinase, cutinase, cyclodextrin glycosyltransferase, deoxyribonuclease, esterase, alpha-galactosidase, beta-galactosidase, glucoamylase, alpha-glucosidase, beta-glucosidase, haloperoxidase, invertase, laccase, lipase, mannosidase, oxidoreductases, pectinolytic enzyme, peptidoglutaminase, peroxidase, phytase, polyphenoloxidase, proteolytic enzyme, ribonuclease, transglutaminase, or xylanase. Products such as Biorem produced by Realco in coordination with Novozyme to target applications in the food and beverage industry exploit a two step cleaning process that invokes use of this kind of multienzyme mixture followed by application of a biocide.

In this industrial sector also, ultrasound has been found a useful tool; for sanitary control, it was found that the combination of chelating agents with ultrasound has been useful for removing selected biofilm-producing pathogens from metal surfaces (Oulahal N, Martial-Gros A, Bonneau M and Blum LJ, "Combined effect of chelating agents and ultrasound on biofilm removal from stainless steel surfaces. Application to "Escherichia coli milk" and "Staphylococcus aureus milk" biofilms", Biofilms, 2004; 1: 65-73, Cambridge University Press.). The efficacy of such ensonification has been shown to exhibit dependency on the frequency and duty cycle of the energy (Nishikawa T, et al., "A study of the efficacy of ultrasonic waves in removing biofilms," Gerontology, Sept 2010; Vol 27, Issue 3: 199-206.).

### Currant Methods for Treating Marine Biofouling

Biofouling occurs worldwide in various industries and one of the most common biofouling sites is on the hulls of ships, where barnacles are often found. A significant problem associated with biofilms on ships is the eventual corrosion of the hull, leading to the ship's deterioration. However, before corrosion occurs, organic growth can increase the roughness of the hull, which will decrease the vessel's maneuverability and increase hydrodynamic drag. Ultimately, biofouling can increase a ship's fuel consumption by as much as 30%. Parts of a ship other than the hull are affected as well: heat exchangers, water-cooling pipes, propellers, even the ballast water. Fishing and fish farming are also affected, with mesh cages and trawls harboring fouling organisms. In Australia, biofouling accounts for about 80% of the pearling industry's costs (Stanczak M, "Biofouling: It's Not Just Barnacles Anymore," CSA Discovery Guide, 2004; http://www.csa.com/discoveryguides/biofoul/overview.php.).

The traditional method of control is to coat exposed surfaces with an anti-fouling compounds. Most of these compounds rely on copper and tin salts that gradually leach from the coating and contaminate the surrounding environment. One of the most widely used coatings to date has been tributyl tin (TBT) which is highly toxic to marine organisms. Since it has been found to have unwanted side-effects on non-target organisms, a world-wide ban on its use was instituted in 2008. The race is on for an environmentally sound alternative (Scottish Association for Marine Science, http://www.sams.ac.uklresearch/departments/microbial-molecular/mmb-project-themes/algal-biofilms.).

Hence, in maritime applications such as shipping, there is an unmet need for viable, cost-effective biofilm remediation.

### Current Methods for Treating Biofims in Heating, Ventilation and Air-Conditioning (HVAC) and Refrigeration Systems

HVAC and refrigeration systems encounter problems associated with biofilms formed on cooling coils, drain pans, and in duct work subjected to water condensation. Biofilm formation on cooling coils diminishes heat exchange efficiency; its growth on other surfaces, including drain pans and duct work, is a source of contamination in the air stream. Conventional methods of addressing biofilms in these applications include maintenance cleaning of coils, duct work and drain pans, use of anticorrosion and antimicrobial coatings on system surfaces, and the exposure of system surfaces to C-band ultraviolet light to break down biofilms and kill pathogens.

### Remediation of Biofilm Contamination in Household Applications

The household products industry is vitally concerned with disinfection of household surfaces, water and plumbing systems, and human hygienic needs. Difficulties associated with killing bacteria attached to these diverse surfaces are well known in this industrial sector and considerable research currently is directed at developing products which kill or remove biofilms.

An innovation in this sector is probiotic-based cleaning. Some versions of these products lay down layers of benign bacteria that successfully compete with pathogenic bacteria for resources on kitchen and bathroom surfaces. Other such products combine enzymes with probiotic bacteria to digest biofilms and dead pathogens. A leading example of this class of products is PIP produced by Chrisal Probiotics of the Netherlands.

The conventional approaches to treatment of biofilm discussed for both medical and industrial applications variously have been unproven, of limited effectiveness, time consuming, costly in cases where large surface areas are involved or surfaces require repeated treatment, and newer concepts have yet to demonstrate effectiveness and scalability to field applications. Hence, there remains an urgent need for more effective and less costly methods to treat biofilms. The present compositions and method offer the prospect of a new standalone approach to biofilm treatment with higher efficacy and lower cost, with additional potential for augmenting certain conventional treatments while reducing the costs of such treatments.

### IV. Summary

Trehalose (a universal general stress response metabolite and an osmoprotectant) can play an important role in the formation and development of microbial biofilm and the specific interactions of trehalose with water can be considered to be one of the most important mechanisms of biofilm formation (see for example: E. W. Petzold et al, "Characterization and Regulation of the Trehalose Synthesis Pathway and Its Importance in the Pathogenicity of Cryptococcus neoformans" in Infection and Immunity, 2006, 74 (10), 5877). Certain enzymes, such as peroxidases and lysozymes (see EP 1068871), *Serratia* peptidases (see US 20110129454) or even certain carbohydrases (see US 6759040), have previously been disclosed to prevent or degrade biofilms. The present compositions and methods have been conceived to target trehalose degradation as a key step in degrading biofilm.

In various embodiments of the compositions and methods, compounds that prevent, degrade, and/or inhibit the formation of biofilms, compositions comprising these compounds, devices exploiting these compounds, and methods of using the same are disclosed.

Because trehalose serves to manipulate hydrogen bonds among water molecules and bacterial cells in the process of forming the biofilm gel, the degradation of trehalose ultimately should result in degradation of the biofilm gel. A class of compounds that degrade trehalose with high specificity, thereby degrading the biofilm matrix gel is disclosed. Specifically, the naturally occurring enzyme trehalase will hydrolyze a molecule of trehalose into two molecules of glucose. The small amount of enzyme trehalase produced in the human body must be augmented with the administration of much larger amounts to treat in vivo biofilm-based infections. Various treatment formulations that incorporate trehalase enzymes and associated delivery mechanisms are detailed for specific types of infections; these include systemic and local treatment protocols. Additionally, trehalase-containing mixtures and associated processes are disclosed to degrade biofilms present on medical instruments and to mitigate biofilm fouling and biofilm-based biocorrosion for industrial applications. For degrading biofilms on medical equipment, trehalase-containing mixtures can be used in concert with other processes, such as ultrasound and ultrasound-assisted enzymatic activity to degrade biofilms. Biofilm prevention approaches comprise the use of trehalase enzymes in surface coatings.

Following is a lexicon of terms and phrases that more particularly define the compositions and methods and support the meaning of the claims:

Time-delayed release - in the context of the present compositions and methods, time-delayed release refers specifically to trehalase (or other compounds) release that occurs at a predetermined approximate time after the trehalase (and in some embodiments, other compounds) in pill, capsule, tablet or other form is ingested orally. Typically, for the present compositions and methods, the time delay means that the initial release of trehalase (or other compounds) will occur in the small intestine, to avoid degradation by naturally occurring proteolytic enzymes in the upper GI tract. Various pre-programmed temporal profiles for release in the small intestine are within the scope of the compositions and methods, such as, for example, linearly increasing or decreasing rates of release with time, or a constant rate of release.

Sustained release - in the context of the present compositions and methods, it refers to the release of trehalase (or other compounds) for applications external to the body. This is a continuous release of trehalase (or other compounds) that is not time-delayed, but is initiated at first opportunity for the purpose of continuous, ongoing exposure of medical device and industrial surfaces to treatment enzymes.

Sufficient for efficacy - pertains to treatment composition amounts and treatment exposure durations adequate to breakdown the gel structure of biofilm for its dispersal and further penetration by antimicrobial agents to treat the target infectious pathogens.

Trehalase - refers to any enzyme selected from the category of trehalase isoenzymes. There are two types of trehalase enzymes found in microorganisms: neutral trehalase (NT) typically found in the cytosol and acid trehalase (AT) found in the vacuoles of the cytosol, either of which type may find application in the present compositions and methods. Further, the number of candidate enzymes is large; as many as 541 model variants (isoenzymes) of trehalase can be found in the Protein Model Portal (http://www.proteinmodelportal.org/), each exhibiting varying potencies in the hydrolysis of trehalose into glucose. The present compositions and methods anticipate a selection from among these isoenzymes that is optimized for the specific biofilm application. For example, the ability to sufficiently purify a given isoenzyme for internal bodily use may favor its selection for this purpose over another isoenzyme that exhibits higher enzymatic activity, but which would be relegated to industrial applications.

Digestive enzymes - are enzymes that break down polymeric macromolecules of ingested food into their smaller building blocks, in order to facilitate their absorption by the body. In the present compositions and methods, treatment formulations comprising trehalase (or other compounds) are disclosed which should: a) avoid degradation by the digestive enzymes naturally occurring in the upper GI tract and b) be combined in time-delayed release form with digestive enzyme supplements to avoid degradation by proteolytic enzymes in such supplements.

Medical devices - comprise devices that are installed either temporarily or permanently in the body and medical instruments that may or may not contact the body, but at least contact tissue or bodily fluids. Examples of temporarily installed medical devices include catheters, endoscopes, and surgical devices. Permanent devices examples include devices such as orthopedic implants, stents, and surgical mesh. Examples of devices used external to the body include stethoscopes, dialysis machines, and blood and urinary analysis instruments. Each of the aforementioned devices exhibit surfaces that are vulnerable to biofilm formation and therefore can benefit from treatment by specific embodiments of the presently disclosed compositions and methods.

Antimicrobials - are substances that kill or inhibit the growth of microorganisms such as bacteria, fungi, or protozoans. Antimicrobials either kill microbes (microbicidal) or prevent the growth of microbes (microbiostatic). Disinfectants are antimicrobial substances used on non-living objects or outside the body.

Other saccharidases (enzymes hydrolyzing saccharides) - include various di-, oligo-, and polysaccharidases.

Living organisms - pertains to the spectrum of living entities from microbes to animals and humans.

GI tract - refers to the gastrointestinal tract; the upper GI tract comprising the mouth, esophagus, stomach, and duodenum, and the lower GI tract comprising the small and large intestines.

Administering via the GI tract - relates to three main alternative treatment delivery methods: first is oral administration in which the treatment compounds are administered via the mouth; for the patients that may not be able to receive treatment by mouth, the second method available is by the naso-gastric tube; and a third method includes delivery by colonic irrigation.

Administering via systemic use - relates to administration of treatment compounds by percutaneous injection, intramuscular injection, intra-venous injection, and venous catheter administration.

### V. Brief Description of the Drawings

FIG. 1a is diagram of the chemical structure of the dissacharide trehalose;
FIG. 1b is a pictorial diagram of the backbone structure of trehalose;
FIG. 2a is a ribbon model pictorial diagram of an enzyme of trehalase derived from Sacharomyces cerevisiae;
FIG. 2b. is a ribbon model pictorial diagram of an enzyme of trehalase derived from Penicillium marneffei;
FIG. 2c is a ribbon model pictorial diagram of an enzyme of trehalase derived from Homo sapiens; and
FIG. 2d is a ribbon model pictorial diagram of an enzyme of trehalase derived from Candida albicans.

### VI. Detailed Description

Since any bacterial biofilm can be defined as a living dynamic structure with spatial and temporal heterogeneity for both, the exopolymer matrix and bacterial microcolonies, the treatment of biofilm-based chronic infections should be aimed at both components simultaneously.

One of the most important survival mechanisms of biofilm-grown microorganisms is the general stress response triggered by a multitude of environmental factors. In the general stress response (ubiquitous in nature), increased production of trehalose (as a general stress response metabolite and an osmoprotectant) plays a dual role as a survival and a defense mechanism.

Trehalose is a disaccharide that is ubiquitous in the biosphere and present in almost all forms of life except mammals. It is one of the most important storage carbohydrates, and may serve as a source of energy and a carbon source for synthesis of cellular components. In various microorganisms, it can also play a structural or transport role, serve as a signaling molecule to direct or control certain metabolic pathways, function to protect cell membranes and proteins against the adverse effects of stresses, such as osmotic stress, heat, cold, desiccation, dehydration, oxidation, and anoxia (Elbein AD, "The metabolism of α,α-trehalose," Adv. Carbohyd. Chem. Biochem., 1974; 30: 227-256.); (Crowe J, Crowe L, and Chapman D, "Preservation of membranes in anhydrobiotic organisms. The role of trehalose," Science, 1984; 223: 209-217.); (Takayama K and Armstrong EL, "Isolation, characterization and function of 6-mycolyl-6'acetyltrehalose in the H37Rv strain of Mycobacterium tuberculosis," Biochemistry, 1976; 15: 441-446.).

Trehalose may be partially responsible for the virulence and antimicrobial resistance properties in various opportunistic and pathogenic microorganisms, including those known to cause chronic infections with biofilm formation in the human body, including: Pseudomonas spp., Bacillus spp., Staphylococci spp., Streptococci spp, Haemophilus influenza, Klebsiella pneumoniae, Proteus spp., Mycobacteria spp., Corynebacteria spp., Enterococci spp., enteropathogenic E. coli, Candida spp., actinomycetes, and other pathogenic yeasts and fungi. As demonstrated in some strains of Candida albicans, interference with the production of trehalose strongly reduces their virulence. Specifically, C. albicans mutants with deleted gene TSP2 which encodes trehalose-6-phosphate phosphatase, one of two enzymes involved in trehalose synthesis, exhibited diminished virulence in an in vivo mouse model of systemic infection and, being grown within in vitro biofilm systems, displayed significantly less biofilm formation than selected non-mutant strains (Coeney T, Nailis H, Tournu H, Van Dick P, and Nelis H, "Biofilm Formation and Stress Response in Candida Albicans TSP2 Mutant," ASM Conference on Candida and Candidiasis, Edition 8, Denver, CO; March 12-17,2006.).

The importance of trehalose, ubiquitous in presence and versatile in function, in microbial life is demonstrated by the fact that all microorganisms can synthesize trehalose intracellularly and/or take it from the environment using multiple synthesis and degradation pathways for trehalose metabolism. The use of less or more of these pathways depends on the genetic program for trehalose utilization in a given bacteria.

The best known and most widely used pathways for intracellular synthesis of trehalose, utilize various nucleoside diphosphate glucose derivatives (ADP-D-glucose, CDP-D-glucose, GDP-D-glucose, TDP-D-glucose and UDP-D-glucose) as glucosyl donors, and a-D-glucose-6-phosphate in a two-step reaction catalyzed by two enzymes: trehalose-6-phosphate synthase (TPS) and trehalose-6-phosphate phosphatase (TPP) (Styrvold OB and Strom AR, "Synthesis, accumulation, and excretion of trehalose in osmotically stressed Escherichia coli K-12 strains: influence of amber suppressors and function of the periplasmic trehalases," J. Bacteriol, 1991; 173(3): 1187-1192. PMID: 1825082). It should be mentioned that some mycobacteria, such as Mycobacterium smegmatis and Mycobacterium tuberculosis, possessing unusual trehalose-6-phosphate synthases, are capable of utilizing all five nucleoside diphosphate glucose derivatives as glucosyl donors (Lapp D, Patterson BW, Elbein AD, "Properties of a trehalose phosphate synthetase from Mycobacterium smegmatis. Activation of the enzyme by polynucleotides and other polyanions," J. Biol. Chem., 1971; 246 (14): 4567-4579.).

Also, trehalose can be synthesized directly from maltose, independently of the presence of the phosphate compounds trehalose-6-phosphate and glucose-6-phosphate. This pathway involves the intramolecular rearrangement of maltose (glucosyl-alphal, 4-glucopyranoside) to convert the 1, 4-linkage to the 1, 1-bond of trehalose; this reaction is catalyzed by the enzyme trehalose synthase and gives rise to free trehalose as the initial product. It is postulated that in Corynebacterium glutamicum this pathway may work in the opposite direction, compensating for the absence of a trehalase enzyme, by converting excess trehalose back into maltose, for reuse as a carbon source (De Smet KA, Weston A, Brown IN, Young DB, Robertson BD, "Three pathways for trehalose biosynthesis in mycobacteria," Microbiology, 2000; 146 (Pt 1): 199-208. PMID: 10658666); (Wolf A, Cramer R, Morbach S, "Three pathways for trehalose metabolism in Corynebacterium glutamicum ATCC 13032 and their significance in response to osmotic stress," Mol Microbiol, 2003; 49(4): 1119-1134. PMID: 12890033.).

In an additional pathway, trehalose can be formed from polysaccharides, such as glycogen or starch, by the action of several enzymes: first, an isoamylase hydrolyzes the α-1, 6-glucosidic linkage in glycogen or the α-1, 4-glucosidic linkages in other polysaccharides, such as starch, to produce a maltodextrin; next, a maltoolgosyl-trehalose synthase (MTS) converts maltodextrin to maltooligosyl-trehalose by forming an α, α-1, 1-glucosidic linkage via intermolecular transglucosylation; and the third enzyme, maltooligosyl-trehalose trehalohydrolase (MTH) hydrolyzes the product to form trehalose and a maltodextrin which becomes shorter by two glucosyl residues. In Corynebacterium glutamicum, which possess three different pathways for trehalose biosynthesis, this is the main route for trehalose biosynthesis (Maruta K, Mitsuzumi H, Nakada T, Kubota M, Chaen H, Fukuda S, Sugumoto T, Kurimota M, "Cloning and sequencing of a cluster of genes encoding novel enzymes of trehalose biosynthesis from thermophilic archaebacterium Sulfolobus acidocaldarius," Biochim Biophys Acta, 1996; 129 (3): 177-181. PMID: 8980629.).

There are several alternative pathways for degradation of trehalose in both unmodified and phosphorylated forms. Unmodified trehalose may be degraded by a hydrolyzing enzyme trehalase (the cytoplasmic trehalase --- TreF, or the periplasmic trehalase - TreA), yielding two β-D-glucose molecules or it may be split by the action of the enzyme trehalose phosphorylase, yielding β-D-glucose-6-phosphate as end product. Trehalose phosphorylase (TP), the key enzyme in several pathways, can also catalyze the reversible synthesis (and degradation) of trehalose from / to a β-D-glucose-1-phosphate and β-D-glucose, or α-D-glucose-1-phosphate and α-D-glucose. Phosphorylated form, trehalose-6-phosphate may be either hydrolyzed by trehalose-6-phosphate hydrolase, yielding β-D-glucose and β-D-glucose-6-phosphate, or degraded by the trehalose-6-phosphate phosphorylase, yielding β-D-glucose-1-phosphate and β-D-glucose-6-phosphate. All end products of the degradation pathways can be metabolized via glycolysis. Trehalose degradation pathways utilizing the phosphorylated form of trehalose, a trehalose-6-phosphate, are found in many bacteria, both Gram-positive and Gram-negative (Helfert C, Gotsche S, Dahl MK, "Cleavage of trehalose-phosphate in Bacillus subtilis is catalyzed by a phospho-alpha-(1-1)-glucosidase encoded by the TreA gene," Mol Microbiol, 1995; 16(1): 111-120. PMID: 7651129.); (Levander F, Andersson U, Radstrom P, "Physiological role of beta-phosphoglucomutase in Lactococcus lactis," Appl Environ Microbiol, 2001; 67(10): 4546-4553. PMID: 11571154.).

Multiple synthesis and degradation pathways for trehalose provide unrestricted opportunities for various microorganisms to utilize trehalose as a universal osmoprotectant in constantly changing environmental conditions. In the live environment of a human body, the bacteria must continuously adapt to temporal and spatial fluctuations in osmolarity of body fluids, even within the range of physiological changes. In osmoadaptation, bacteria constitutively use the universal mechanism of uptake and release of osmotically active compounds (osmolytes). Bacteria adapt to the conditions of increased external osmolarity by importing charged ions from the environment, and importing or synthesizing compatible solutes. Upon a shift to a low-osmolarity media, the excretion of these osmoprotectants is required to restore normal turgor and prevent the cells from bursting. The pathways for import and efflux of compatible solutes include PTS system, ABC transporters, mechanosensitive channels, and porins (Berrier CM, Besnard M, Ajouz B, Coulombe A, and Ghazi A, "Multiple mechanosensitive ion channels from Escherichia coli, activated at different thresholds of applied pressure," J. Membr. Biol., 1996; 151: 175-187.); (Bremer R and Kraemer R, "Coping with osmotic challenges: osmoregulation through accumulation and release of compatible solutes in bacteria," pp. 79-97. In G. Storz and R.Hengge-Aronis (ed.), Bacterial stress responses. 2000; ASM Press, Washington, D.C.); (Chang G, Spencer R, Lee AT, Barclay MT, and Rees DC, "Structure of the MscL homolog from Mycobacterium tuberculosis: a gated mechanosensitive ion channel," Science, 1998; 282: 2220-2225.); (Morbach S and Kraemer R, "Body shaping under water stress: osmosensing and osmoregulation of solute transport in bacteria," ChemBioChem, 2002; 3: 384-397.).

Compatible solutes are small, zwitterionic, highly soluble organic molecules, which include diverse substances, such as amino acids (proline,glutamate), amino acid derivatives (glycine betaine, ectoine), and sugars (trehalose and sucrose), that are thought to stabilize proteins and lead to the hydration of the cell (Steator RD and Hill C, "Bacterial osmoadaptation: the role of osmolytes in bacterial stress and virulence," FEMS Mocrobiol. Rev., 2002; 26: 49-71.). Various bacteria may prefer different osmolytes taken from the environment, but all of them constitutively utilize trehalose as a universal osmoprotectant. For example, E. coli and Vibrio Cholerae in human GI tract prefer glycine betaine, but its synthesis relies on an external supply of proline, betaines, or choline which may not be readily available in the environment or significantly reduced in the deeper layers of microbial biofilm. When these compounds are not available, a cell can achieve a moderate level of osmotic tolerance by accumulation of glutamate and trehalose (Styrvold OB, Strom A R, "Synthesis, accumulation, and excretion of trehalose in osmotically stressed Escherichia coli K-12 strains: influence of amber suppressors and function of periplasmic trehalase," J Bacteriol, 1991; 173 (3): 1187-1192. PMID: 1825082.); (Kapfhammer D, Karatan E, Pflughoeft KJ, and Watnik PI, "Role for Glycine Betaine Transport in Vibrio cholera Osmoadaptation and Biofilm Formation within Microbial Communities," Applied and Environmental Microbiology, July 2005: 3840-3847.).

As demonstrated in laboratory-grown bacteria, the first adaptive response to osmotic stress comprises both the increased uptake rate and the amount of cytosolic potassium, followed by the accumulation of glutamate and synthesis of trehalose (Dinnbier U, Limpinsel E, Schmid R, and Bakker EP, "Transient accumulation of potassium glutamate and its replacement by trehalose during adaptation of growing cells of Escherichia coli K-12 to elevated sodium chloride concentrations," Arch. Microbiol., 1988; 150: 348-357.); (McLaggan D, Naprstek J, Buurman ET, and Epstein W, "Interdependence of K+ and glutamate accumulation during osmotic adaptation of Escherichia coli," J. Biol. Chem., 1994; 269: 1911-1917.); (StromAR and Kaasen I, "Trehalose metabolism in Escherichia coli: stress protection and stress regulation of gene expression," Mol. Microbiol., 1993; 8: 205-210.). The time-dependent (10 to 60 minutes) alterations in the proteome of E. coli (grown under aerobic conditions) in response to osmotic stress, demonstrated upregulated genes for synthesis of both trehalose and cytosolic trehalase - TreF (trehalose-degrading enzyme with regulatory properties) in the middle phase(10 to 30 minutes) and the long phase (30 to 60 minutes) of bacterial adaptation to hyperosmotic stress, with the trehalase - TreF synthesis genes being already upregulated in the early phase of adaptation (0 to 10 minutes) (Weber A, Kögl SA, and Jung K, "Time-Dependent Proteome Alterations under Osmotic Stress during Aerobic and Anaerobic Growth in Escherichia coli," Journal of Bacteriology, Oct. 2006: 7165-7175. doi: 10.1128/JB.00508-06.).

Synthesis and/or transport of compatible solutes are time-dependent and energy-consuming processes, especially for anaerobically grown cells, and are costly for bacteria. Therefore, in any given bacteria, the preference for choosing compatible solutes in the face of osmotic stress will favor substances (or their precursors) that are always available in the environment, can be reused in cell metabolism, provide fast and flexible response to continuously changing osmolarity, and for which transport and synthesis are less energy consuming. Trehalose seems to fulfill all these requirements as a universal stress response metabolite and an osmoprotectant.

Trehalose is a stable dissacharide with glycosidic bond [O-α-D-Glucopyranosyl-(1-1)-α-D-glucopyranoside] formed from a condensation between the hydroxyl groups of the anomeric carbons of two molecules of glucose, preventing them from interacting with other molecules and thereby rendering trehalose among the most chemically inert sugars (Birch GG, "Trehaloses," Adv. Carbohydr. Chem. Biochem., 1963; 18: 201-225.); (Elbein AD, "The metabolism of alpha, alpha-trehalose," Adv. Carbohydr. Chem. Biochem., 1974; 30: 227-256.). The flexible glycosidic bond, together with the absence of internal hydrogen bonds, yields a supple molecule, but this glycosidic bond does not break easily: the 1kcal/mol linkage is highly resilient, enabling the trehalose molecule to withstand a wide range of temperature and pH conditions (Pava CL and Panek AD, "Biotechnological applications of the disaccharide trehalose," Biotechnol. Annu. Rev., 1996; 2: 293-314.). Because of the unusual glycosidic bond between the anomeric carbons (1-1), there are no more accessible carbons for further polymerization, so that trehalose exists only as a disaccharide, being rather distributed as disaccharide molecules in the gel-like matrix of biofilm, influencing its density via interaction between trehalose and water molecules. Intermolecular hydrogen bonds (H bonds), the strongest of intermolecular forces, are central to trehalose interaction with water. Specifically, such bonds modify the structure of water surrounding trehalose molecules and account for the self-aggregation phenomena of trehalose molecules observed in molecular dynamic simulations and supported by experimental studies.

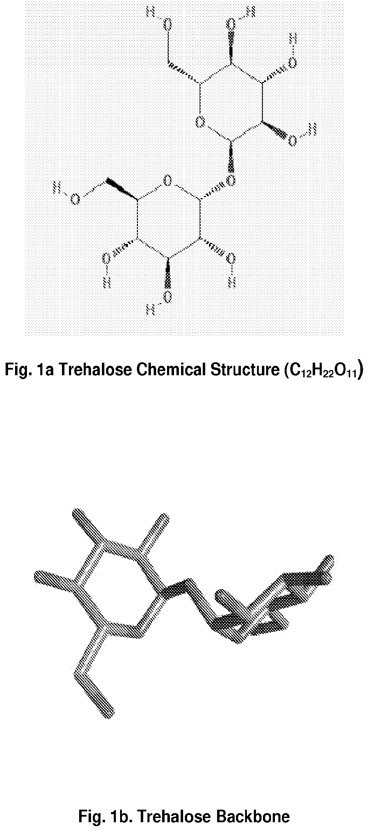

The chemical structure of trehalose is depicted in Figure 1a indicating an alpha-linked disaccharide formed by an α,α-1,1-glucosidic bond between two α-glucose units. The backbone structure of this enzyme is shown in Figure 1b depicting the two planes established by the glucose units.

Trehalose has been determined to capture water through extensive solvation. Water molecules are arranged in a solvation complex around trehalose molecules, with water associating with trehalose functional groups through H bond formation; at infinite dilution, the solvation number approaches 15 (the highest among all disaccharides). This relatively large hydration number supports a potent ability to restructure water at minimum aqueous concentrations of trehalose and contribute to gelation phenomena. With respect to water restructuring behavior, trehalose enhances the hydrogen bonding between water molecules by approximately 2%. This is sufficient to destructure the pure water tetrahedral network in conformity with a restructuring imposed by trehalose clusters. Stronger, more linear, and better optimized H bonds are formed between water molecules, while weaker bonds are relegated to trehalose-water interactions (Sapir L and Harries D, "Linking Trehalose Self-Association with Binary Aqueous Solution Equation of State," J. Phys. Chem. B, 2011; 115: 624-634.).

Trehalose self-associates in aqueous solutions in a concentration dependent manner to form clusters of increasing size, until finally forming percolating, infinitely connected, clustering networks (at concentrations of 1.75 M and higher), affecting the dynamic properties of the solution. The lack of intramolecular hydrogen bonds in trehalose, compared with other disaccharides (sucrose, maltose, isomaltose), accounts for its higher tendency to aggregate, thereby already affecting the dynamic properties of water at lower trehalose concentrations (Lebret A, Bordat P, Affouard F, Descamps M, Migliardo FJ, Phys. Chem. B, 2005; 109: 11046.); (Lebret A, Affouard F, Bordat P, Hedoux A, Guinet Y, and Descamps M, Chem. Phys., 2008; 345:267.); (Peric-Hassler L, Hansen HS, Baron R, and Hunenberger PH, "Conformational properties of glucose-based disaccharides investigated using molecular dynamics simulations with local elevation umbrella sampling," Carbohydr. Res., 2010; 345: 1781.)

In a ternary mixture of protein (lysozyme), sugar, and water, at a moderate concentration of 0.5 M, trehalose can cluster around the protein, thereby trapping a thin layer of water molecules with modified solvation properties, playing the role of a "dynamic reducer" for solvent water molecules in the hydration shell around the protein. A remarkable conformational rigidity of the trehalose molecule due to anisotropic hydration (very little hydration adjacent to the glycosidic oxygen of trehalose), provides stable interactions with hydrogen-bonded water molecules; trehalose makes an average of 2.8 long-lived hydrogen bonds per each step of molecular dynamic simulation compared with the average of 2.1 for the other sugars (Lins RD, Pereira CS, and Hunenberger PH, "Protein-Trehalose Interactions in Aqueous Solution," Proteins, 2004; 55: 177.); (Choi Y, Cho KW, Jeong K, and Jung S, "Molecular dynamic simulations of trehalose as a 'dynamic reducer' for solvent water molecules in the hydration shell," Carbohydr Res., June 12, 2006; 341(8): 1020-1028.).

A simulated ternary mixture of lipid membranes composed of DPPC (dipalmitoylphosphatidylcholine) in contact with an aqueous solution of trehalose, shows that trehalose molecules cluster near membrane interfaces, forming hydrogen bonds, both between trehalose molecules and with the lipid headgroups (Pereira CS, Hunenberger PH, "The effect of trehalose on a phospholipid membrane under mechanical stress," Biophys. J., 2008; 95: 3525.); (Sum AK, Faller R, and de Pablo JJ, "Molecular simulation study of phospholipid bilayers and insights of the interactions with disaccharides," Biophys. J., 2003; 85: 2830.). Trehalose may compete with water binding to both carbonyls and phosphates in cell membranes, forming the OH bridges that are stronger than the H-bonds of water with those groups, and the displacement of water is compensated with the insertion of sugar. Trehalose, a dimer of glucose with the ability to form at least 10 hydrogen bonds, inserts in a lipid interface nearly normal to the lipid bilayer plane and can decrease water activity in the cell membrane up to 70% at a concentration of trehalose as low as 0.1 mM. The insertion of trehalose, replacing water simultaneously at the carbonyls and the phosphates, does not cause the surface defects in the cell membrane with respect to hydrated lipids (Pereira CS and Hunenberger PH, "The effect of trehalose on a phospholipid membrane under mechanical stress," Biophys. J., 2008; 95:3525.); (Sum AK, Faller R, and de Pablo JJ, "Molecular simulation study of phospholipid bilayers and insights of the interactions with disaccharides," Biophys. J., 2003; 85: 2830.); (Villareal M, Diaz SB, Disalvo EA, Montich G, Langmuir, 2004; 20: 7844.).

As a result of water displacement, trehalose may affect the cell surface potential and hence cell aggregation and attachment to surfaces. There can be at least two mechanisms for these phenomena. First, the magnitude of cell surface potential can be modulated by trehalose displacement of water in its attachment to cell membrane phospholipids and carbonyl compounds. Second, this same displacement of water (in a non-uniform manner) can lead to heterogeneity of surface potential, also imparting the adhesion properties (Poortinga AT, Bos R, Norde W, and Busscher HJ, "Electric double layer interactions in bacterial adhesion to surfaces," Surface Science Reports, 2002; 47: 1-31.); (Disalvo EA, Lairion F, Martini F, Almaleck H, Diaz S, and Gordillo G, "Water in Biological Membranes at Interfaces: Does it Play a Functional Role?," An. Asoc. Quim. Argent., 2004; V.92 n. 4-6 Buenos Aires ago. / dic.).

### Trehalose and biofilm formation

Based on the unique properties of trehalose as a universal general stress response metabolite and an osmoprotectant, and the specific features of its interactions with water (which comprises up to 95% of biofilm matrix), trehalose can be one of the most important components of microbial biofilm, and its specific interactions with water can be considered to be one of the most important mechanisms of biofilm formation.

The success of any bacteria as a pathogen in a human body will depend on its ability to adapt to a new environment, adhere to the surface and remain there under protective covering of the biofilm, establishing itself as a biofilm-based chronic infection. Since the formation of microbial biofilm can be seen as a continuous process of adaptation of a microorganism to its environment, trehalose and its interactions with water can play an important role in all stages of biofilm development.

From the first moment, when a microorganism enters the human body in a planktonic form, it is subjected to various stresses (first of all, osmotic stress) and undergoes the general stress response with the production of trehalose, which in its initial interactions with water begins the process of microbial biofilm formation. In this initial stage, trehalose facilitates adhesion of planktonic bacteria to surfaces by various means: as a result of its interaction with water and the lipid headgroups at the cell membrane interfaces, it decreases the microbial cell surface potential and enhances the bacterial cell aggregation, initial adsorption and attachment to the surfaces, both biotic and abiotic. Also, trehalose favors the bacterial cell aggregation and attachment to various surfaces by forming a hydration layer with modified solvation properties around the bacterial cell and reducing the dynamic properties of water in this layer (and up to the 3-rd and 4-th hydration layers), thus slowing down the bacterial cell movement. In addition, trehalose self-associates in aqueous solution in a concentration dependent manner to form clustering networks, affecting the dynamic properties of the solution. Through extensive solvation, trehalose has a potent ability to restructure water in the solution and enhance the hydrogen bonding between water molecules, thus contributing to the gelation phenomena and the biofilm formation.

During the next stage of the biofilm development (the formation of bacterial colonies and the maturation of biofilm), the bacteria will continuously produce trehalose as a general stress response metabolite and an osmoprotectant in response to constantly varying environmental conditions, such as increased cell density, nutrients limitations, and waste products accumulation in the biofilm. Then, the continuous trehalose - water interactions, with attraction of new water molecules and further restructuring of water, will result in formation of new layers of the biofilm and gradually increased biofilm volume. During this stage, bacteria will release into the biofilm matrix various extracellular substances, including specific proteins (adhesins, matrix interacting factors), compatible solutes, metabolic end- or by-products, such as polysaccharides, lipids, phospholipids, and the detritus from aging and lysed cells, which will contribute to the formation of the tertiary structure of the biofilm, stabilization of the biofilm architecture, thickening of the biofilm matrix, and increased density of the biofilm.

As the biofilm ages, the amount of trehalose in the superficial layers of the biofilm can decrease due to higher accumulation of trehalose in the deeper layers adjacent to the bacterial cells, so that the trehalose restructuring effect on water, the strengthening effect on the hydrogen bonds between water molecules, and the aggregation forces between the bacterial cells gradually diminish and favor the sloughing off of the superficial layers of the biofilm, and dissemination of the pathogenic bacteria to the new places.

At any stage of the biofilm development, bacteria will respond to any environmental assault on the biofilm, including the use of various disinfectants and antimicrobials, by additional production of trehalose as a general stress response metabolite and an osmoprotectant, that will result in further increase of the biofilm gel matrix volume and density, preventing the penetration of harmful substances into the biofilm.

Trehalose was detected along with other sugars, di- and polysaccharides in the laboratory-grown microbial biofilms in laboratory studies, mostly aimed at either evaluating the effect of various nutrients on biofilm formation, or analyzing the content of the biofilm exopolymer matrix. But to date, no specific conclusions have been made in these studies in regard to either the importance of trehalose as a specific constituent of the biofilm or the possible role of trehalose and its interactions with water in the mechanisms of microbial biofilm development.

For example, trehalose was detected in a small amount (3%), along with glycerol (5%), mannitol (18%), and glucose (74%), in the monosaccharide-polyol fraction of the aerial-grown hyphae of the Aspergillus fumigatus biofilm; all hexoses and polyols were found intracellularly in the same proportion as extracellularly (Beauvais A, Schmidt C, Guadagnini S, Roux P, Perret E, Henry C, Paris S, Mallet A, Prevost M, and Latge JP, "An extracellular matrix glues together the aerial-grown hyphae of Aspergillus fumigates," Cellular Microbiology, 2007; 9(6): 1588-1600.). In another example, biofilm development on stainless steel by Listeria monocytogenes (the most common biofilm-producing pathogen in the food industry), was enhanced by the presence of mannose or trehalose as nutrients in the growth media, with trehalose being superior to mannose in constant biofilm production during 12 days of incubation at 21 degrees C (Kim KY and Frank JF, "Effect of nutrients on biofilm formation by Listeria monocytogenes on stainless steel," Journal of food protection, 1995; 58(1): 24-28.). In another study, the formation of a structurally and metabolically distinctive biofilm by Streptococcus mutans (the most common pathogen in dental biofilms), was enhanced by the combination of sucrose and starch, compared with sucrose alone, in the presence of surface-adsorbed salivary a-amylase and bacterial glucosyltransferases, with upregulation of genes associated with maltose uptake/transport and fermentation/ glycolysis (Klein MI, DeBaz L, Agidi S, Lee H, Xie G, Lin AH, Hamaker BR, Lemos JA, and Koo H, "Dynamics of Streptococcus mutans Transcriptome in Response to Starch and Sucrose during Biofilm Development," PLoS ONE, 2010; 5(10): 1-13.). In the next study, the yeasts from hydrocarbon-polluted alpine habitats (Cryptococcus terreus - strain PB4, and Rhodotorula creatinivora - strains PB7 and PB12) synthesized and accumulated glycogen (both acid- and alkali-soluble) and trehalose during growth in culture media, containing either glucose or phenol as a sole carbon and energy source, with higher biofilm formation by both strains of Rhodotorula creatinivora (Krallish I, Gonta S, Savenkova L, Bergauer P, and Margesin R, "Phenol degradation by immobilized cold-adapted yeast strains of Cryptococcus terreus and Rhodotorula creatinivora," Extremophiles, 2006; 10(5): 441-449.).

In contrast to the previous results, the laboratory-grown wild type Enterococcus faecalis formed strong biofilm in the presence of maltose or glucose in the growth media, and formed very little amount of biofilm in medium containing trehalose (Creti R, Koch S, Fabretti F, Baldassarri L, and Johannes H, "Enterococcal colonization of the gastro-intestinal tract: role of biofilm and environmental oligosaccharides," BMC Microbiology, 2006; 6: 660-668.).

Since trehalose is the most abundant disaccharide in yeasts and fungi, the biofilm matrix of any biofilm-based yeast or fungal infections, and/or multispecies biofilms which include yeasts and/or fungi, can be more resistant to penetration by antimicrobials. In clinical observations, it has been demonstrated that biofilms with mixed bacterial and Candida infections or biofilm-based Candida spp. chronic infections were difficult to treat, even with applied enzymatic formulations that included amylases, various saccharidases (but no specific enzymes for trehalose degradation were included), peptidases, proteinases, lipases, and fibrinolytic enzymes.

### Enzyme trehalase

Trehalose can be degraded by the highly specific enzyme trehalase (alpha, alpha-trehalose-glucohydrolase), yielding two molecules of glucose on hydrolysis, and this process appears to be important, perhaps essential, in the life functions of various organisms, including yeasts, bacteria, and insects (Nwaka S and Holzer H, "Molecular biology of trehalose and trehalases in the yeast, Saccharomyces cerevisiae," Prog. Nucleic Acid Res. Mol. Biol., 1998; 58: 197-237.). Enzyme trehalase (α.α-trehalase; α,α-trehalose-1-C-glucohydrolase, EC 3.2.1.28) has been reported to be present in many micro-and macroorganisms, including animals and plants, but in most cases neither the functions nor the properties of this important enzyme have been studied (Elbein AD, "The metabolism of α,α-trehalose," Adv. Carbohyd, Chem. Biochem, 1974; 30: 227-256.); (Elbein AD, Pan YT, Pastuszak I, and Carroll D, "New insights on trehalose: a multifunctional molecule," Glycobiology, 2003; Vol. 13, No 4: 17R-27R.).

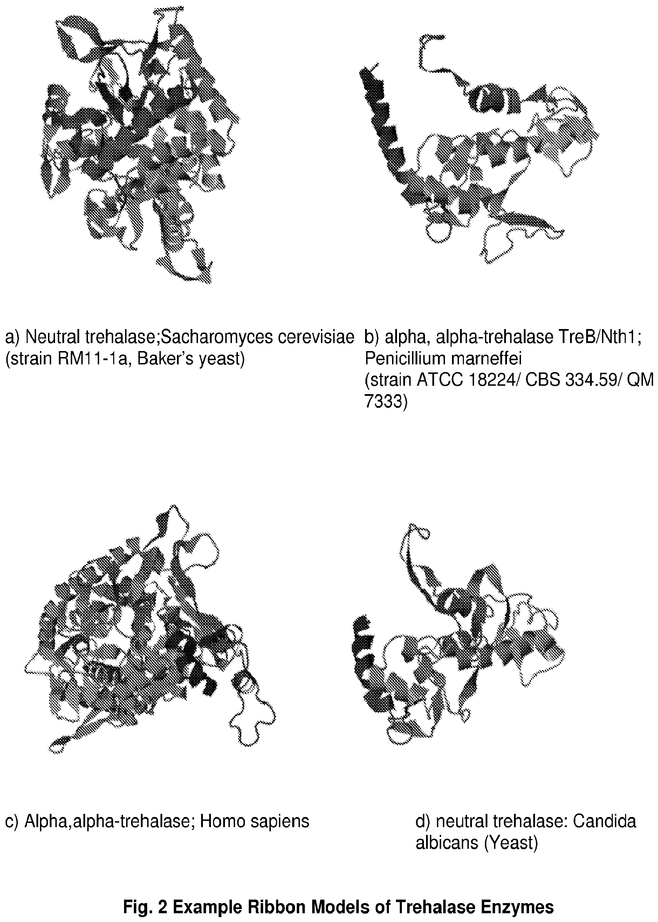

As many as 541 model variants of this enzyme can be found in the Protein Model Portal (http://www.proteinmodelportal.org/). A few of these models corresponding to different enzyme variants (isoenzymes) are shown in Figures 2a through 2d.

In lower forms of life (yeasts, fungi, bacteria), there are two main types of trehalase enzyme: neutral trehalase (NT) and acid trehalase (AT), which are encoded by two different genes - NTH1 and ATH1 respectively. Most of the trehalase activity in these microorganisms, comes from the neutral trehalase, located in the cytosol, with the pH optimum of about 7, highly specific for trehalose as the substrate, and inactive on cellobiose, maltose, lactose, sucrose, raffinose, and mellibiose; this enzyme has also a specific regulatory function (App H and Holzer H, "Purification and characterization of neutral trehalase from the yeast ABYS1 mutant," J. Biol. Chem., 1989; 264: 17583-17588.). The acid or vacuolar trehalase has a pH optimum of 4.5 and is also very specific for trehalose as the substrate, showing no activity with cellobiose, maltose, lactose, sucrose, and mellibiose; this enzyme acts in the periplasmic space where it binds exogenous trehalose to internalize it and cleave it in the vacuoles to produce free glucose (Mittenbuhler K and Holzer H, "Purification and characterization of acid trehalases from the yeast SUC2 mutant," J. Biol. Chem., 1988; 263: 8537-8543.); (Stambuk BU, de Arujo PS, Panek AD, and Serrano R, "Kinetics and energetics of trehalose transport in Saccharomyces cerevisiae," Eur. J. Biochem., 1996; 237: 876-881.).

The activities of both trehalases are low in yeast cells growing exponentially, but high during stationary phase growth after glucose has been depleted (Winkler K, Kienle I, Burgert M, Waner JC, and Holzer H, "Metabolic regulation of the trehalose content of vegetative yeast," FEBS Lett., 1991; 291: 262-272.). ATH1 deletion mutant of the yeast S. cerevisiae cannot grow in the medium with trehalose as the carbon source, but a Candida utils mutant strain is able to utilize extracellular trehalose as carbon source despite of the lack of AT activity. Various bacteria, such as E. coli, have trehalases that may function as part of the uptake system to supply glucose to the PTS, as well as be involved in metabolism of trehalose as an osmoregulator (Horlacher R, Uhland K, Klein W, Erhmann M, and Boos W, "Characterization of a cytoplasmic trehalase of Escherichia coli," J. Bacteriol., 1996; 178: 625-627.).

In the plant kingdom, enzyme trehalase is ubiquitous, though its substrate trehalose is rare in vascular plants. No clear role has been demonstrated for trehalase activity in plants, but it has been suggested that plant trehalase could play a role in the defense against parasites and other pathogenic organisms, or it could take a part in the degradation of trehalose derived from the plant-associated bacteria (Muller J, Wiemken A, and Aeschbacher R, "Trehalose metabolism in sugar sensing and plant development," Plant Sci., 1999; 147: 37-47.); (Muller J, Aeschbacher RA, Wingler A, Boller T, and Wiemken A, "Trehalose and trehalase in Arabidopsis," Plant Physiol., 2001; 125: 1086-1093.).

Though disaccharide trehalose is not known to be present in mammals, the enzyme trehalase is found in mammals, including humans, both in the kidney brush border membranes and in the intestinal villae membranes; the role of trehalase in kidney is still not clear, but in the intestine its function is to hydrolyze ingested trehalose (Dahlqvist A, "Assay of intestinal disaccharidases," Anal. Biochem., 1968; 22: 99-107.); (Ruf J, Wacker H, James P, Maffia M, Seiler P, Galand G, Kiekebusch A, Semenza G, and Mantei N, "Rabbit small intestine trehalase. Purification, cDNA cloning, expression and verification of GPI-anchoring," J. Biol. Chem, 1990; 265: 15034-15040.); (Yonemaya Y and Lever JE, "Apical trehalase expression associated with cell patterning after inducer treatment of LLC-PK monolayers," J. Cell. Physiol., 1987; 131: 330-341.). In contrast to other enzymes of trehalose metabolism, only α,α-trehalase is present in humans: produced by the glands of Lieberkuhni in the small intestine, it is a constituent of the intestinal juice along with other specific saccharidases, such as maltase, sucrase-isomaltase complex, Beta-glycosidase-lactase (Mayes PA, "Carbohydrates of physiologic significance," In: Harper's Biochemistry, 25th ed, 2000, pp. 149-159, Appleton & Lange, Stamford, CT.); (Rodwell VW and Kennelly PJ, "Enzymes: General Properties; Enzymes: Kinetics," In: Harper's Biochemistry, 25th ed, 2000, pp. 74-102, Appleton & Lange, Stamford, CT.). As with all other disaccharidases, trehalase remains attached to the brush border of the enterocyte in the intestinal lumen while the catalytic domain is free to react with the substrate. There is little free trehalase activity in the intestinal lumen; most activity is associated with small "knobs" on the brush border of the intestinal epithelial cells. A small fraction (approximately 0.5%) may be absorbed by passive diffusion, as shown for other disaccharides, in patients with trehalase deficiency (van Elburg RM, Uil JJ, Kokke FTM, Mulder AM, van dr Broek WGM, Mulder CJJ, and Heymans HSA, "Repeatability of the sugar-absorption test, using lactulose and mannitol, for measuring intestinal permeability for sugars," J. Pediatr. Gastroenterol. Nutr., 1995; 20: 184-188.). Traces of trehalase activity have been found also in the renal cortex, plasma, urine, liver and bile, although function of the enzyme in these locations is not clear yet; it is likely that trehalase in the urine and bile can be incidental to its presence in the kidney and liver (Eze LC, "Plasma trehalase activity and diabetes mellitus," Biochem Gen., 1989; 27: 487-495.).

Biochemical properties of the human enzyme α,α--trehalase include:
- high specificity for the substrate (disaccharide trehalose)
- method of activation - direct contact with the substrate (trehalose)
- optimal conditions for activity - in the range of pH between 5.0 and 7.0 (similar to the other disaccharidases)
- end product of action - 2 molecules of glucose
- heat sensitivity - as a glycosylated protein is probably similar to the other disaccharidases
- catalytic efficiency --- high due to the high specificity for the substrate trehalose
- coenzymes or metal ions for activity - not needed
- co-variants of enzyme - unknown

The function of the human α,α-trehalase enzyme is to hydrolyze ingested disaccharide trehalose into glucose. Trehalase deficiency is a known metabolic condition, when the body is not able to convert disaccharide trehalose into glucose; people with this deficiency experience vomiting, abdominal discomfort and diarrhea after eating mushrooms, with most cases appear to be inherited in an autosomal recessive manner (Kleinman RE, Goulet O, Mieli-Vergani G, Sherman PM, In: Walker's Pediatric Gastrointestinal Disease: Physiology, Diagnosis, Management, 5-th edition, 2008); (Semenza, G., Auricchio, S., and Mantei, N. In: The Metabolic & Molecular Bases of Inherited Disease; 8-th ed., 2001; Chapter 75: Small Intestinal Disaccharidoses. McGraw-Hill, New York.). Isolated intestinal trehalase deficiency is found in approximately 8% of Greenlanders; it is not infrequent among Finns, but is believed to be rare elsewhere. The low (2%) incidence of isolated trehalase enzyme deficiency was described in the populations from the USA, UK, and mainland Europe (Bergoz R, Valloton MC, and Loizeau E, "Trehalase deficiency," Ann. Nutr. Metab., 1982; 26: 191-195.). In the UK, from 400 patients investigated for suspected malabsorption, 369 (92%) had normal intestinal histology on biopsy, with the normal range of trehalase at 4, 79 - 37, 12 U/g protein; 31 (8%) patients with villous atrophy had a diagnosis of coeliac disease and significantly reduced activity of disaccharidases, including trehalase, with recovered function of all enzymes (except lactase) after treatment with a gluten-free diet; the authors concluded that there is no basis for routine determination of trehalase activity in the population of the UK (Murray IA, Coupland K, Smith JA, Ansell ID, Long RG, "Intestinal trehalase in a UK population: Establishing a normal range and the effect of disease," Br. J. Nutr., 2000; 83(3): 241-245.). In Belgium, in intestinal biopsy samples from 200 patients with abdominal symptoms and diarrhea, total α.α-trehalase deficiency (0-12 U/g mucosa) was detected in 18 (9%) cases, partial deficiency (3-12 U/g mucosa) - in 39 (19.5%) cases, and only 4 patients (2%) presented selective α,α-trehalase deficiency with otherwise normal other disaccharidases; these data suggested that α,α-trehalase deficiency can be more common than it is believed (Buts JP, Stilmant C, Bernasconi P, Neirinck C, De Keyser N, "Characterization of alpha, alpha-trehalase released in the intestinal lumen by the probiotic Saccharomyces boulardii," Scandinavian Journal of Gastroenterology, 2008; 43 (12): 1489-1496.).

The importance of trehalase was demonstrated in certain pathologic conditions, including birth defects and genetic abnormalities: low or absent intestinal trehalase isozyme was detected in the sample of amniotic fluid from a fetus with anal imperforation, whereas a higher than normal level of renal trehalase activity was found in amniotic fluid from a fetus with polycystic kidney disease (Elsliger MA, Dallaire L, Potier M, "Fetal intestinal and renal origins of trehalase activity in human amniotic fluid," Clin Chim Acta, July, 16, 1993; 216(1-2): 91-102.). Also, low intestinal trehalase enzyme level was detected in amniotic fluid on amniocentesis in 14 pregnant women at 1 in 4 risk for a child with cystic fibrosis, screened at the 18-th week of gestation; and in two terminated at the 19-th week cases, histochemical lesions characteristic of cystic fibrosis were seen in exocrine glands, including the pancreas and intestinal mucosa of both fetuses, and the total protein content in the meconium of these fetuses was also significantly higher than in the controls (Szabo M, Teichmann F, Szeifert GT, Toth M, Toth Z, Torok O, Papp Z, "Prenatal diagnosis of cystic fibrosis by trehalase enzyme assay in amniotic fluid," Article first published online: 23 APR 2008; DOI: 10.1111/j. 1300-0004. 1985.tb01211.x.). The trehalase enzyme assay in amniotic fluid was recommended as a genetic test for prenatal diagnosis of cystic fibrosis.

Since ingestion of large quantities of foods containing trehalose is not common worldwide, the real frequency of trehalase deficiency in various populations around the world is mostly unknown. However, it should be noted, that over the last two decades, in addition to natural sources of trehalose in the food (mostly, mushrooms, algae, baker's yeasts), it has been approved in some countries, including the USA, as an additive in the preparation of dried, frozen, and processed food, and as a moisture retainer in various products (including ice cream, and baked goods), with no requirements for labeling of this constituent in prepared food or other products (Abbott PJ and Chen J, WHO Food Additives Series 46: Trehalose. International Programme on Chemical Safety. Accessed Feb 4, 2010, available at:
http://www.inchem.org/documents/jecfa/jecmono/v46je05.htm.).

The amount of enzyme trehalase normally produced for digestion and utilization of exogenous trehalose is appropriate for healthy people, but is far less than what is needed for people with biofilm-based chronic infections, especially for individuals with trehalase enzyme deficiency. Therefore, the use of enzyme trehalase, along with other enzyme formulations and antimicrobials (including antibiotics), can greatly enhance the effectiveness of various treatment protocols for biofilm-based chronic infections.

Therefore, at least one basis for the presently disclosed compositions and methods is the addition of enzyme trehalase, highly specific to the hydrolysis of the trehalose constituent of microbial biofilms, to treatment protocols for biofilm-based chronic infections in order to increase the effectiveness of existing treatment modalities.

Enzyme trehalase can be obtained from natural sources (plants, yeasts, fungi), can be manufactured in various forms (powder, liquid, gel, tablets, and capsules), delivered to any specific location in the body where biofilm is the issue (mostly mucosal linings, oral cavity, respiratory tract, urinary tract, and GI tract), can be used alone or in concert with other enzymes, and can be used to control biofilms on medical devices and industrial fluid conduits. However, to date no available medical/health scientific information shows evidence of this enzyme as a component of any prescription drugs, OTC products, or nutritional supplements, either alone or in enzymatic formulations, as well as a component for biofilm treatment on medical devices. Also, there is no available information about using of the enzyme trehalase for the biofilm problem in industrial settings.

### Embodiments for the treatment of biofilm-based infections

To increase effectiveness of existing protocols for biofilm-based chronic infections in the human body, in accordance with a first aspect of the invention, trehalase enzyme can be used alone or in combination with other enzymes either in direct application to the sites of infectious biofilm (directly accessible mucosal linings of the respiratory tract, GI-tract, genito-urinary tract, eyes, skin, open wounds, etc.) and/or as a systemic enzyme alone or included in multi-enzyme formulations for addressing biofilm-based infections in directly inaccessible (or hardly accessible) sites of infection and in the bloodstream.

In direct application to the sites of bacterial biofilm, trehalase enzyme should be used in a multi-step procedure, starting with application of trehalase (alone or in combination with other saccharidases) with an exposition time sufficient to adequately degrade the biofilm matrix, followed in a second step by application of combination of other enzymes to break down proteins and lipids (proteolytic, fibrinolytic, and lipolytic enzymes) over a corresponding appropriate exposition time; and the third step in this procedure should be an application of antimicrobials specific for the infection(s) involved, or polymicrobial antibiotics.

As a systemic enzyme, trehalase should be used alone or in combination with other saccharidases as time-delayed release substance(s), or be included in multi-enzyme formulations as time-delayed release constituent(s) to avoid early degradation by proteolytic enzymes in the upper GI tract (stomach and duodenum) and/or by proteolytic enzymes in administered formulations, and finally be released in the small intestine for further absorption. In this way, trehalase can be supplied for direct absorption and distribution via the bloodstream to hardly accessible "niches" of biofilm-based infections, for example, on the inner lining of the blood vessels, in bones, joints, on implanted medical devices, etc.).

Also within the scope of the present compositions and methods, are methods of insuring that enzymes, other than trehalase and other saccharidases mentioned, administered for health maintenance or medical reasons, are protected from co-administered proteolytic enzymes, other co-administered compounds, and proteolytic enzymes naturally occurring in the upper GI tract. Such methods of protection include creating time-delayed release formulations of the enzymes to be protected whether they are orally admininstered alone or in combination with other enzymes. The time delays can be established so that release of the enzymes to be protected occurs in the small intestine. Also, differential time delays can be established for protected enzymes and any co-administered proteolytic enzymes to avoid deleterious interactions of these compounds. In conventional digestive or systemic enzyme formulations currently on the market, contained enzymes typically are not protected from proteolytic degradation.

### Upper Respiratory Tract

The major biofilm-forming species of pathogens affecting the upper respiratory tract include Haemophilus influenzae, Klebsiella pneumoniae, Pneumococcus, Streptococcus spp., Staphylococcus spp., Pseudomonas aeruginosa, Candida spp., and Aspergillus spp. For these types of biofilm-based infections in the upper respiratory tract (chronic sinusitis, rhinosinusitis, tonsillitis, pharyngitis, and otitis media), trehalase enzyme can be used alone or with other saccharidases for direct application to the sites of infectious biofilms on mucosal linings in liquid form as a saline-based solution for instillations, irrigations, and sprays, as well as in gel, ointment, and powder forms.

Local treatment should comprise a multi-step procedure with the first step being the application of trehalase (alone or with other saccharidases) with adequate exposition time, with the second step being the application of proteolytic, fibrinolytic, and lipolytic enzymes over a corresponding appropriate exposition time, and the final step comprising application of antimicrobials specific to the infection present or polymicrobial antibiotics with longer exposition time to address specific infectious pathogens. Local treatment can be reinforced by using a systemic enzyme formulation (including trehalase in a time-delayed release form) and systemic antibiotics, preferably with polymicrobial activity.

For Pseudomonas aeruginosa infection, an additional enzyme, alginate lyase (highly specific for the polysaccharide alginate - an important constituent of Pseudomonas aeruginosa biofilm), can be added to trehalase or trehalase in combination with other saccharidases in a local application to the site of biofilm-based infection. For Streptococcus spp. infections, an additional enzyme, dextranase (highly specific for the dextrans - oligosaccharides produced by Streptococcus spp., which facilitate microbial adhesion to the mucosal surfaces and biofilm formation), can be added to trehalase or trehalase in combination with other saccharidases in a local application to the site of biofilm-based infection. Local application of trehalase (alone or with other saccharidases) can be reinforced by using a systemic enzyme formulation (including trehalase or trehalase with other saccharidases in a time-delayed release form) and systemic antibiotics, preferably with polymicrobial activity.

### Otitis Media

For otitis media with or without effusion, treatment should include a systemic enzymes formulation (with trehalase or trehalase with other saccharidases in time-delayed release form) along with systemic antibiotics. This treatment can be reinforced with local treatment in a multi-step procedure: initial application of trehalase alone or with other saccharidases (for example, enzyme alginate lyase - highly specific for polysaccharide alginate in Pseudomonas aeruginosa biofilm, or enzyme dextranase - highly specific for oligosaccharides dextrans in Streptococcal biofilm), followed by the application of proteolytic, fibrinolytic, and lipolytic enzymes, and finally, antibiotics to the lining of the nasal cavity to address the infection spread to the middle ear from the nasal and sinus cavities. Delivery to the inner ear can be by a nasal instillation with a pathway through the Eustachian tube into the middle ear.

For otitis media with effusion and installed tympanic tubes, the abovementioned systemic and local treatments should be reinforced by an additional step: the installed tympanic tubes can be covered inside with trehalase, other saccharidases (including, for example, highly specific alginate lyase and dextranase), and antimicrobials specific to pathogens present or polymicrobial antibiotics.

### Lower respiratory Tract

Treatment of biofilm-based infections in the lower respiratory tract, should include: a) systemic enzymes (with trehalase alone or trehalase and other saccharidases in time-delayed release form) along with systemic antibiotics; b) brochoalveolar or whole lung lavage in a multi-step procedure, including the use of trehalase alone, or trehalase with other saccharidases (for example, alginate lyase, dextranase) in a saline-based solution in the first step, followed by proteolytic, fibrinolytic, and lipolytic enzymes in the second step, and antibiotics in the third step; c) nasal and sinus instillations (in a multi-step procedure) of trehalase alone or trehalase with other saccharidases (preferably, specific to existing pathogens), followed by proteolytic, fibrinolytic, and lipolytic enzymes, and finally by antibiotics.

Additional contributing factors to chronic biofilm-based infectious conditions are: genetic trehalase enzyme deficiency (a rare genetic disease listed by NIH Genetic and Rare Diseases Information Center), genetic trehalase enzyme deficiency in individuals with cystic fibrosis; and artificial trehalase deficiency due to widespread use of trehalose in the food industry as an approved additive in the preparation of dried food and as a moisture conservant in many foods, such as an ice cream and baked goods.

Taking into account genetic trehalase deficiency in cystic fibrosis patients, uncontrolled consumption of trehalose in food is a favorable factor for thick biofilm formation on the mucosal lining of the upper and lower respiratory tracts in such individuals. Pseudomonas aeruginosa, in symbiosis with other bacteria and fungi, exploits this environment with production of polysaccharide alginate and increased production of trehalose, resulting in a thick polymicrobial biofilm, which is almost impossible to eradicate with long-term antibiotic therapy alone (although such therapy can support the patient). To address this polymicrobial biofilm at any stage of its development, treatment should include: a) the use of trehalase or trehalase with other saccharidases in time-delayed release form as the constituents of systemic enzyme formulations; b) brochoalveolar or whole lung lavage in a multi-step procedure, including the use of trehalase alone, or trehalase and other saccharidases (for example, alginate lyase, dextranase) in a saline-based solution in the first step, followed by proteolytic, fibrinolytic, and lipolytic enzymes in a saline-based solution in the second step, and antibiotics in the third step; and c) continuous use of systemic antibiotics. This treatment can be reinforced by nasal and sinus instillations (in a multi-step procedure) of trehalase alone or trehalase with other saccharidases (preferably, specific to present pathogens), followed by proteolytic, fibrinolytic, and lipolytic enzymes, and finally, by antibiotics.

### Native Valve Endocarditis (NVE), Infectious Endocarditis, and Line Sepsis

A preferred treatment protocol for NVE, Infectious Endocarditis, and Line Sepsis as blood stream infections, should include systemic administration of trehalase alone or in combination with other saccharidases (preferably, specific to present pathogens) in time-delayed release form; proteolytic, fibrinolytic, and lipolytic enzymes; and antibiotics directed to specific infectious agents, or polymicrobial antibiotics. The typical organisms involved in these biofilm-mediated infectious conditions include Streptococci spp, Enterococci spp., Pneumococcus, Staphylococci spp.(both coagulase positive and negative), gut bacteria, and fungi (most often, Candida albicans and Aspergillus spp.). Because all these pathogens gain access to the bloodstream primarily via the oropharynx, GI-tract, and genito-urinary tract, systemic treatment of NVE, Infectious Endocarditis, and Line Sepsis should be reinforced by local treatment of those infections at the sites of origin, including the previously described multi-step procedure (with application of trehalase, other enzymes, and antimicrobials) if the sites of origin represent biofilm-based infections.

### Chronic Bacterial Prostatitis (CBP) and Urinary Tract Infections (UTI)

Use of systemic enzymes with included trehalase alone or trehalase with other saccharidases in time-delayed release form, and antimicrobials, will address the presence of biofilm-based chronic infections in both CBP and UTI. For local treatment of UTI via bladder instillation, a method after the fashion of the multi-step procedure disclosed above for treating mucosal linings should be employed: the first step being the application of trehalase (alone or with other saccharidases) with adequate exposition time; the second step being the application of proteolytic, fibrinolytic, and lipolytic enzymes over a corresponding appropriate exposition time; and the final step comprising application of antimicrobials (antibiotics) with longer exposition time to address specific infectious pathogens. For local treatment of CBP, again, the same multi-step procedure should be used, but with higher antibiotic concentrations delivered directly to the biofilm within the prostatic ducts by instillation means (via a medical device such as a catheter).

### GI Tract Infections

GI tract infections are characterized by polymicrobial biofilm communities along with helmintic infections (nematodes are known to produce trehalose). For treating microbial biofilms in the upper GI tract, formulations of digestive enzymes should include trehalase alone or trehalase with other saccharidases. For treatment of biofilm-based infections in the lower GI tract, formulations of digestive enzymes should include trehalase alone or trehalase with other saccharidases in time-delayed release form to avoid early degradation by proteolytic enzymes in the upper GI tract or by proteolytic enzymes in the same formulations. Optionally, the multi-step local treatment (with trehalase alone or with other saccharidases) disclosed above for treating infectious biofilm on mucosal linings can be used, especially for treating infectious biofilms located in the lower intestinal tract (as local colonic treatment). In addition to the use of enzymes (including trehalase), local and systemic antimicrobials directed against specific microorganisms, including possible symbiotic infections, parasites, or protozoa should be administered.

### Dental and Periodontal Diseases

The two groups of bacteria responsible for initiating caries, including Streptococcus mutans and Lactobacillus (known to possess multiple pathways for biosynthesis of trehalose), have direct access to high concentrations of orally ingested simple sugars and other saccharides, as well as those produced by the action of salivary amylase on ingested carbohydrates, that favors the increased synthesis of trehalose and formation of the biofilm. Enzyme trehalase alone or in combination with other saccharidases can be used for prevention of dental caries by inhibiting the formation of bacterial biofilms on the teeth and surrounding tissue surfaces.

Periodontal disease is a classic biofilm-mediated condition that is refractory to treatment by antimicrobials alone. Applied treatments, which include trehalase alone or in combinations with other saccharidases, can be both preventive and curative. Trehalase (alone or with other saccharidases) can be combined with antimicrobials in oral application for treatment of periodontal diseases and/or during a professional dental cleaning procedure. Also, the multi-step local treatment, including the application of trehalase alone or with other saccharidases, followed by the application of proteolytic, fibrinolytic, and lipolytic enzymes, and finally by the application of antimicrobials, as disclosed above for treating infectious biofilm on mucosal linings, can be used as a curative method for periodontal biofilm-based infections. Since the bacterial biofilm is the essence of the dental plaque, the use of trehalase alone or with other saccharidases in the mouthwash or gel form can diminish the formation of the dental plaque, and in prolonged use in combination with antimicrobials can gradually degrade and eliminate the existing bacterial biofilms.

For dental surgery, trehalase formulations can serve as prophylaxis against biofilm-based infections. Trehalase can be used in conjunction with antimicrobial substances in pre- and post-operative dental surgery. Additionally, it can be combined with the other materials commonly used to treat teeth in endodontics, such as dental cements.

A prophylactic application of trehalase in dental hygiene includes its use in mouthwashes, toothpastes, dental floss, and chewing gum. Trehalase can be combined with conventional non-alchohol-containing mouthwashes (to avoid alcohol-induced denaturation of the enzyme); such compositions also typically include menthol, thymol, methyl salicylate, and eucalyptol. Trehalase inclusion in toothpaste is straightforward, without chemical interaction with components of conventional toothpaste; typical toothpaste formulations comprise: abrasive 10-40%, humectant 20-70%, water 5-30%, binder 1-2%, detergent 1-3%, flavor 1-2%, preservative 0.05-0.5% and therapeutic agent 0.1-0.5%. Impregnation of dental floss fibers with trehalase is analogous to the inclusion of flavorings used in dental floss materials such as silk, polyamide, or Teflon. Finally, trehalase (alone or with other saccharidases) can be included in a chewing gum composition to prevent the formation of bacterial biofilms and dental plaques, as well as to treat oral biofilm-based infections in treatment protocols with antimicrobials.

### Mitigation of Ingestion of Excess Trehalose by Susceptible Individuals

Owing to its unique chemical structure, trehalose remains stable under low pH conditions, even at elevated temperatures. Over the last two decades, the agri-food industry has introduced the use of trehalose in many foodstuffs as a food stabilizer, sweetener, and a moisture retainer, since the high stability of trehalose enables the original product characteristics to be retained even after heat processing, freezing, and prolonged storage. Usually, the product labeling does not indicate the presence or amount of this food additive. Patients exhibiting biofilm-based infections, especially those with genetic trehalase enzyme deficiency, can be at increased risk upon consumption of the dietary trehalose, as the excess of this sugar either can be used by the gut bacteria for local GI tract biofilm formation or, being absorbed in the gut and presented via circulation directly to the infectious organisms in various locations, can contribute to the development and persistence of the biofilm-based chronic infections in various sites of the human body. For mitigation of these negative events, trehalase can be used as an enzyme alone (in a time-delayed release form), or can be added to existing formulations of digestive and systemic enzymes (in the same time-delayed release form) for individuals at increased risk upon consumption of dietary trehalose.

### Embodiments for the Treatment of Biofilm-Based Contamination of Medical Devices

The methods for treatment of biofilm-contaminated medical devices comprise two categories, preventive and curative. The preventive methods of the present compositions and methods rely on altering the composition of device surfaces by incorporating trehalase enzyme, whereas curative methods exploit temporary exposure of these surfaces to treatment formulations based on solitary trehalase or trehalase in concert with other compounds and protocols.

### Preventive Methods

In accordance with a second aspect of the invention, use of coatings (both delayed release and non-delayed release) and enzyme immobilization on surfaces are two methods that can prevent biofilm growth on medical devices. Simple (non-delayed release) coatings can be applied to metal, polymer, and fabric surfaces to provide a brief, initial exposure of treatment enzyme. Delayed release coatings can release an enzyme into the surrounding environment over time to degrade biofilm, ultimately depleting the initial amount of coating-contained enzyme. In contrast to these coatings, an enzyme immobilized on a surface can act as a permanent, reusable catalyst, providing the potential for ongoing degradation of biofilm.

Treatment coatings can be applied to porous surfaces such as those of fabric-based prosthetic heart valve cuffs and surgical mesh used for hernia repair and nonporous surfaces such as metal and polymer medical device surfaces. Delayed release coatings that discharge trehalase enzymes or trehalase enzymes in combination with other agents (such as antimicrobials) over time offer the prospect of prophylactic action against the formation of biofilms. These coatings are especially useful on the biofilm-vulnerable surfaces of medical devices and for use on temporary and permanent bodily implants. Conventional examples of delayed release coatings include enzymes embedded in surface porosity either pre-existing or specially-created at the surface, surface-attached microencapsulated enzymes, and dissolvable coatings overlaying the enzyme on the surface. The structure and composition of these conventional coatings, the methods of their adhesion to the device or implant surface, and the mechanisms of time release of agents of interest are well known in the prior art and can be modified to exploit the use of trehalase in the present compositions and methods.

Trehalase can be immobilized (as discussed below in greater detail with respect to curative methods) on the biofilm-vulnerable surfaces of medical devices. A substantial body of work is devoted to the details of enzyme immobilization on polymer and metal surfaces (ex.:Drevon GF, "Enzyme Immobilization into Polymers and Coatings," PhD Dissertation, University of Pittsburgh, 2002). Immobilization of trehalase on the surface of medical devices can even be combined with other materials of antimicrobial nature such as silver and copper or with biofilm attachment preventives like Bacticent™ KB. Trehalase can be immobilized on a compound that serves as a support structure and this support structure compound can be bound to device surfaces. This insures that trehalase enzymatic activity is preserved by avoiding direct interaction of trehalase with the device surfaces. From among the numerous candidate support structure compounds, a choice can be optimized with respect to maintaining the enzyme activity of trehalase while achieving high binding affinity to the device surfaces.

Trehalase-based treatment coatings, both delayed release and non-delayed release, as well as immobilized trehalase can be used on the interior and exterior surfaces of central venous and urinary catheters, and the biofilm-vulnerable surfaces of endoscopes and implants of various types including orthopedic implants. Further, trehalase can be combined with antimicrobial compounds in coatings or immobilized states on devices to improve effectiveness. The impregnation of surgical mesh or fabrics with trehalase is yet another application. A foremost example is a method to prevent biofilm formation and growth on prosthetic heart valves by impregnating the fabric sewing cuff with trehalase before attachment of the cuff to the heart valve assembly. Additionally, the heart valve assembly can be covered with an immobilized trehalase coating.

The surfaces of implantable and bodily-inserted devices are targets of both the immune response and bacterial colonization, a so-called "race for the surface" (Gristina A, "Biomedical-centered infection: microbial adhesion versus tissue integration," Clinical Orthopedics and Related Research, 2004, No. 427, pp. 4-12.). In the case of the immune response acting first, macromolecule adhesion and general inflammatory action can lead ultimately to the enclosure of the device surface by a nonvascular fibrous capsule which further can support bacterial colonization and biofilm formation. If bacterial colonization occurs before overt immune response, biofilm can form immediately adjacent to the device surface. Since both the accumulation of host cells at the device surface and bacterial colonization of the surface have initial macromolecule adhesion in common, defeat of such adhesion in vivo is synergistic with use of trehalase to impede biofilm formation.

For this purpose, trehalase can be combined with new coatings that offer the promise of deterring macromolecule adhesion to synthetic surfaces. Among examples are Semprus Sustain™ technology, a polymeric approach to harnessing water molecules at device surfaces to impede macromolecule attachment, Optichem® antifouling coating with microporosity excluding macromolecule contact with the protected device surface, and zwitterionic coatings (Brault ND, Gao C, Xue H, Piliarik M, Homola J, Jiang S, Yu Q, "Ultra-low fouling and functionalizable zwitterionic coatings grafted onto SiO2 via a biomimetic adhesive group for sensing and detection in complex media," Biosens Bioelectron., 2010 Jun 15, 25(10): 2276-2282.) that suggest the prospect of defeating protein adhesion through the exploitation of periodic reversal of polarity in the surface coating. Delayed release coatings which include trehalase can be used in concert with macromolecule-repellant coatings in various modes. For example, trehalase time release sites can be established with adequate density within the confines of a macromolecule-repellant coating. Alternatively, disparate coatings can be interleaved in various geometries both parallel and perpendicular to the device surface.

### Curative Methods

Methods of the present compositions and methods that address degradation and removal of biofilms and associated pathogens from surfaces, not in accordance with the present invention, involve various soak (immersion) and rinse protocols. Solutions of trehalase enzymes, with other compounds such as other enzymes, chelating agents, and stabilizers are anticipated. In a preferred embodiment of a soak solution, the present inventive use of trehalase enzymes to degrade the biofilm gel matrix can be viewed as an important addition to enzyme mixtures found in such products as the aforementioned Biorem. Immersive exposure to trehalase-based soak solutions can be followed by exposure to biocidal treatments, as are well known in the prior art, for elimination of pathogens. Rinse and soak solutions containing trehalase should be maintained at the temperature of maximum enzyme activity. Also, soak and immersion durations should be made sufficient for effectiveness.

A preferred method of solution-based treatment comprises the following multi-step procedure:
1. creating a first treatment solution taken from the group comprising: a) trehalase alone in aqueous or saline solution and b) trehalase with other saccharidases in aqueous or saline solution,
2. creating a second treatment solution taken from the group comprising: a) proteolytic enzymes in aqueous or saline solution and b) fibrinolytic enzymes in aqueous or saline solution,
3. creating a third treatment solution taken from the group comprising: a) biocides in aqueous or saline solution, b) antibiotics, specific to the infectious agents present in aqueous or saline solution, or c) polymicrobial antibiotics in aqueous or saline solution,
4. flushing (or rinsing) or immersing the surface under treatment with these solutions in the sequence given.

The exposure time for the treated surface should be sufficient for effectiveness and such solution treatments should take place in a manner that avoids exposure of trehalase to proteolytic enzymes.

This multi-step procedure can be applied to treatment of central venous and urinary catheters, endoscopes, contact lenses and lens cases, dialysis system components, dental unit water lines, and other medical devices that can be subjected to immersion, rinse, or fluid injection. In the case of dialysis systems, various surfaces that contact biological fluids must be disinfected. However, some surfaces can be immersed in treatment solutions with the option of ultrasound-assisted cleaning, other surfaces are not immersible and simply must be soaked and flushed with treatment solutions. Also, for dialysis system components and dental unit water line treatment, the aforementioned third solution additionally can contain chelating agents and enzyme stabilizers.

An alternative avenue of trehalase delivery involves immobilization of the enzyme by attachment to a support structure compound of some kind. In contrast to immobilization on device surfaces, as discussed above, trehalase can be immobilized on a support structure compound that is in liquid suspension for use as a treatment liquid. Immobilization of the enzyme can permit its extended presence and repeated use in catalysis. Additionally, it can increase the enzyme's catalytic efficiency and thermal stability based on the specifics of its attachment to the support structure. There are five general categories of such immobilization: a) adsorption, b) covalent binding, c) entrapment, d) encapsulation, and e) cross-linking (Walker JM, Rapley R, and Bickerstaff GF, "Immobilization of Biocatalysts" in Molecular Biology and Biotechnology, 4th edition, edited by J. M. Walker and R. Rapley, RSC Publishing, 2007). All such mechanisms are within the scope of the present compositions and methods. In the delivery of trehalase enzymes to biofilm, some immediate implementations of immobilization are envisioned herein. For example, enzymes can be covalently bound to microspheres, as discussed below, or encapsulated in liposomes after the fashion of U. S. Patent 7,824,557 (which discloses the use of antimicrobial-containing liposomes to treat industrial water delivery systems). These delivery mechanisms can be incorporated by uptake into the biofilm matrix to provide sustained exposure to trehalase enzymes.

The feasibility of trehalase immobilization is underscored by examples of trehalase immobilization for various non-treatment purposes that can be found in the recent research literature. For analytical purposes, Bachinski et al. demonstrated the immobilization of trehalase on aminopropyl glass particles by covalent coupling. In this work, it was shown that the enzyme retained its catalytic activity (N. Bachinski, A. S. Martins, V. M. Paschoalin, A. D. Panek, and C. L. Paiva, "Trehalase immobilization on aminopropyl glass for analytical use," Biotechnol Bioeng., 1997 Apr 5, 54(1): 33-39.). For reactor reuse, trehalase has been immobilized on chitin as well (A. S. Martinsa, D. N. Peixotoa, L. M.C. Paivaa, A. D. Paneka and C. L.A. Paivab, "A simple method for obtaining reusable reactors containing immobilized trehalase: Characterization of a crude trehalase preparation immobilized on chitin particles," Enzyme and Microbial Technology, February 2006, Volume 38, Issues 3-4, Pages 486-492.). The present compositions and methods includes immobilization of enzyme trehalase on support structures that have particular affinity for biofilms. U. S. Patent Application No. 20060121019 discloses the covalent and non-covalent attachment of biofilm degrading enzymes to "anchor" molecules that have an affinity for the biofilm. Moieties cited as having a known affinity for biofilms included Concanavalin A, Wheat Germ Agglutinin, Other Lectins, Heparin Binding Domains, Elastase, Amylose Binding Protein, Ricinus communis agglutinin I, Dilichos biflorus agglutinin, and Ulex europaeus agglutinin I.

A preferred method of using immobilized trehalase in liquid treatment comprises the same solution-based multi-step procedure outlined above, but using immobilized trehalase in aqueous or saline suspension. Likewise, the method is similarly applicable to treatment of the same categories of medical devices disclosed above.

As mentioned, ensonification of the surface to be treated can be employed to augment the removal of biofilms concomitantly with soak and rinse solutions. Apart from this traditional use of ultrasound for biofilm removal, an additional modality that is within the scope of the present compositions and methods is the use of ultrasound-assisted enzymatic activity. The introduction of a low energy, uniform ultrasound field into various enzyme processing solutions can greatly improve their effectiveness by significantly increasing their reaction rate. The process is tuned so that cavitation does not result in reduction in enzyme activity, but rather significant increase. This is achieved by proper uniformity of ensonification and use of lower power levels.

It has been established that the following specific features of combined enzyme/ultrasound action are critically important: "a) the effect of cavitation is several hundred times greater in heterogeneous systems (solid-liquid) than in homogeneous, b) in water, maximum effects of cavitation occur at ~50 degC, which is the optimum temperature for many industrial enzymes, c) cavitation effects caused by ultrasound greatly enhance the transport of enzyme macromolecules toward substrate surface and, d) mechanical impacts, produced by collapse of cavitation bubbles, provide an important benefit of "opening up" the surface of substrates to the action of enzymes." (Yachmenev V, Condon B, Lambert A, "Technical Aspects of Use of Ultrasound for Intensification of Enzymatic Bio-Processing: New Path to "Green Chemistry"," Proceedings of the International Congress on Acoustics, 2007). Enzyme reaction rates can be increased by more than an order of magnitude. In an example of specific enzyme application, alpha amylase reaction rates were increased with the use of ultrasound (Zhang Y, Lin Q, Wei JN, and Zhu HJ, "Study on enzyme-assisted extraction of polysaccharides from Dioscorea opposite," Zhongguo Zhong Yao Za Zhi. 2008 Feb, 33(4): 374-377.). For ultrasound-assisted enzyme-based treatment, the solution-based multi-step treatment previously disclosed, can be modified to include ensonification of enzyme-containing treatment solutions and surfaces under treatment.

### Embodiments to Address Industrial Biofilms

There are numerous industrial biofilm treatment approaches that can be enabled by the use of trehalase enzymes. These approaches involve both creation of appropriate mixtures of trehalase enzymes with other compounds and methods for delivery of these mixtures to the sites of biofilm presence.

With respect to treatment mixtures, trehalase enzymes can be used alone in solution or added to compounds that maintain the optimum pH range (buffer compositions) and metallic ion concentrations that can maximize the hydrolysis rate of trehalose. Additionally, one or more trehalase enzymes can be added to compositions of dispersants, surfactants, detergents, other enzymes, anti-microbials, and biocides that are delivered to the biofilm in order to achieve synergistic effects. Trehalase can be used as a pretreatment in a protocol involving other biofilm treatment compounds or methods that could decompose trehalase or diminish its enzymatic (catalytic) activity.

Also, trehalase can be immobilized on substrate compounds in liquid suspensions, as discussed above, for use in industrial treatments, where the substrate compound may have an affinity for the target of treatment.

In accordance with a third aspect of the invention, for oil pipelines, an oil-water emulsion containing trehalase enzyme mixtures will provide a dosing opportunity to the biofilms within the pipeline. These emulsion-borne mixtures can include free trehalase enzymes or immobilized enzymes as well as additional conventional treatment compounds such as biocides, surfactants, detergents, and dispersants as are well known in the prior art.

A specific treatment embodiment for pipelines involves the exploitation of annular liquid flow geometries. The annular flow pattern of two immiscible liquids having very different viscosities in a horizontal pipe (also known as "core-annular flow") has been proposed as an attractive means for the pipeline transportation of heavy oils since the oil tends to occupy the center of the tube, surrounded by a thin annulus of a lubricant fluid (usually water) (Bannwar AC, "Modeling aspects of oil-water core-annular flows," Journal of Petroleum Science and Engineering Volume 32, Issues 2-4, 29 December 2001, Pages 127-143.). A thin water film can be introduced between the oil and the pipe wall to act as a lubricant, giving a pressure gradient reduction. In 8-inch diameter pipes, it has been shown that, under certain conditions, it is possible to use very thin water films. For crude oils with viscosities exceeding 2000 mPas, stable operation has proved feasible with as little as 2 % water (Oliemans RVA, Ooms G, Wu HL, Duijvestijn A, "Core-Annular Oil/Water Flow: The Turbulent-Lubricating-Film Model and Measurements in a 2-in. Pipe Loop," Middle East Oil Technical Conference and Exhibition, 11-14 March 1985, Bahrain.). In an embodiment of the present compositions and methods to address delivery of trehalase-containing solutions to the interior of oil pipelines, the thin water film is replaced by a trehalase-containing aqueous solution. This trehalase solution will be a flowing annular layer immediately adjacent to the inner surface of the pipeline.

Another embodiment of the compositions and methods addressing pipelines comprises the exploitation of magnetic force to deliver trehalase to the target treatment sites within pipelines. Specifically, trehalase can be immobilized on a support structure compound that exhibits either magnetic or preferably ferromagnetic properties. When this immobilized trehalase is released into pipeline flow, a magnetic field exterior to the pipeline can be used to guide and retain the immobilized trehalase in the target vicinity on the interior of the pipeline. The magnetic field can be generated by magnetic or electromagnetic means well known in the prior art. Optimization of this embodiment could include spatial and temporal variation of the generated magnetic field to achieve appropriate concentration of trehalase at treatment sites in the presence of fluid flow. Residual magnetism induced in the pipeline wall can be diminished by methods well known in the prior art.

Not in accordance with the invention, dry dock removal of hull biofouling material including biofilms can use aqueous solutions containing trehalase enzymes in rinse and/or soak protocols. Application of trehalase containing hydrogels to ships' hulls is another means of ensuring sustained exposure of the biofilm for hydrolysis of the trehalose component of the biofilm matrix. This can be done prior to or at the time of biocide application. Further, the application of biofilm preventive coatings that incorporate immobilized trehalase enzymes to marine surfaces is a candidate approach. The solution-based, multi-step treatment discussed for medical device treatment can be used in this marine application or modified to use gel delivery of treatment compounds instead of aqueous or saline solutions.

In accordance with a fourth aspect of the invention, for HVAC systems the solution-based multi-step treatment method can be used as stated for certain components such as cooling coils and drain pans, or modified so that treatment compounds can be fed into HVAC ductwork in the form of aerosols.

Candidate industrial biocides for use with trehalase enzyme-based treatments include popular industrial biocide products on the market such as Ultra KleenTM manufactured by Sterilex Corp., Hunt Valley, MD , the active ingredients of which comprise:
n-Alkyl(C14 60%, C16 30%, C12 5%, C18 5%) dimethylbenzylammonium chloride; and
n- Alkyl(C 12 68%, C 14 32%) dimethylethylbenzylammonium chloride.

Another example is SWG Biocide manufactured by Albermarle Corp., Baton Rouge, LA, the active ingredients of which comprise sodium bromosulfamate and sodium chlorosulfamate. Candidates may also be found among the wider generic categories of industrial biocides comprising: glutaraldehyde, quaternary ammonium compounds (QACs), blends of Gut and QACs, Amine salts, Polymeric biguanide, benzisothiazolone, blend of methyl isothiazolones, and acrolein (Handbook of Biocide and Preservative Use, Edited by H. W. Rossmoore, Chapman and Hall, 1995).

Not in accordance with the invention, for treatment of biofilms associated with food processing, storage, and transport systems, conventional enzyme treatments can be augmented with the use of trehalase (A long list of conventional candidate enzymes was disclosed above.). This can be done in the context of the solution-based multi-step procedure. The present compositions and methods include use of trehalase with any such enzymes that are not proteolytic. Also, ultrasound-assisted enzyme-based cleaning is applicable with the use of trehalase.

Biofilms are found in the household environment on many surfaces including the inside surfaces of plumbing and drainpipes, on the surfaces of sinks, bathtubs, tiling, shower curtains, shower heads, cleaning sponges, glassware, toothbrushes, and toilets. Not in accordance with the invention, solutions containing trehalase can be used alone or in proper combination with other biofilm treatment products tailored to the applicable surface. For example, certain compounds used for plumbing treatment would be inadmissible for treating toothbrushes. The aforementioned solution-based multi-step procedure easily can be applied to many household surfaces with the exception of the internal surfaces of plumbing. Again, there is the caveat that proteolytic enzymes and other compounds that degrade the enzymatic activity of trehalase are not present at the same time as trehalase.

## Claims

1. A composition for use in the treatment of biofilm-based infections in direct application to a site of infectious biofilm of humans, the composition comprising trehalase.

2. Use of a composition to prevent biofilm formation and growth on biofilm vulnerable surfaces of medical devices suitable for exposure to bodily fluids and tissues, by contacting said surfaces with the composition, the composition comprising trehalase that is immobilized on said surfaces of said medical devices.

3. Use of a composition in oil pipelines to prevent and treat microbial biofilms in the oil pipelines, the composition being an oil-water emulsion comprising trehalase with or without biocides.

4. Use of a multi-step procedure of first, second and third treatment compositions in the treatment of microbial biofilms in heating, ventilation, and air conditioning (HVAC) systems, the first composition comprising trehalase with or without other saccharidases, the second composition comprising proteolytic enzymes, fibrinolytic enzymes, lipolytic enzymes, buffer agents, surfactants, detergents, chelating agents or enzyme stabilizers, and the third composition comprising biocides, whereby the compositions are used such that exposure of the first composition to the potentially degrading second or third compositions is avoided.

## Patentansprüche

1. Zusammensetzung zur Anwendung in der Behandlung von Biofilm-basierten Infektionen bei direktem Auftrag auf eine Stelle von infektiösem Biofilm des Menschen, wobei die Zusammensetzung Trehalase aufweist.

2. Anwendung einer Zusammensetzung zur Prävention von Biofilmbildung und - Wachstum an Biofilm-anfälligen Oberflächen medizinischer Geräte, die zur Exposition mit Körperflüssigkeiten und Geweben durch Kontaktieren der Oberflächen mit der Zusammensetzung geeignet sind, wobei die Zusammensetzung Trehalase aufweist, die an diesen Oberflächen dieser medizinischen Geräte immobilisiert ist.

3. Anwendung einer Zusammensetzung in Ölpipelines zur Prävention und Behandlung von mikrobiellen Biofilmen in den Ölpipelines, wobei die Zusammensetzung eine Öl-Wasser Emulsion ist, die Trehalase mit oder ohne Bioziden aufweist.

4. Anwendung eines Mehrschritt-Verfahrens von ersten, zweiten und dritten Behandlungszusammensetzungen in der Behandlung von mikrobiellen Biofilmen in Heiz-, Lüftungs- und Luftklimasystemen (HVAC), wobei die erste Zusammensetzung Trehalase mit oder ohne anderen Saccharidasen aufweist, wobei die zweite Zusammensetzung proteolytische Enzyme, fibrinolytische Enzyme, lipolytische Enzyme, Puffermittel, oberflächenaktive Stoffe, Reinigungsmittel, Chelatbildner oder Enzymstabilisatoren aufweist, und wobei die dritte Zusammensetzung Biozide aufweist, wodurch die Zusammensetzungen so angewendet werden, dass eine Exposition der ersten Zusammensetzung zu den potentiell degradierenden zweiten oder dritten Zusammensetzungen vermieden wird.

## Revendications

1. Composition utilisable dans le traitement d'infections à base de biofilm en application directe sur un site de biofilm infectieux d'humains, la composition comprenant de la tréhalase.

2. Utilisation d'une composition pour empêcher la formation et la croissance de biofilm sur des surfaces vulnérables aux biofilms de dispositifs médicaux appropriés pour une exposition à des fluides et tissus corporels, par mise en contact desdites surfaces avec la composition, la composition comprenant de la tréhalase qui est immobilisée sur lesdites surfaces desdits dispositifs médicaux.

3. Utilisation d'une composition dans des oléoducs pour empêcher et traiter des biofilms microbiens dans les oléoducs, la composition étant une émulsion huile-eau comprenant de la tréhalase avec ou sans biocides.

4. Utilisation d'une procédure à plusieurs étapes de première, deuxième et troisième compositions de traitement dans le traitement de biofilms microbiens dans des systèmes de chauffage, ventilation et conditionnement d'air (CVCA), la première composition comprenant de la tréhalase avec ou sans autres saccharidases, la deuxième composition comprenant des enzymes protéolytiques, des enzymes fibrinolytiques, des enzymes lipolytiques, des agents tampons, des agents tensioactifs, des détergents, des agents chélatants ou des agents stabilisateurs d'enzymes, et la troisième composition comprenant des biocides, moyennant quoi les compositions sont utilisées de telle sorte qu'une exposition de la première composition aux deuxième ou troisième compositions potentiellement dégradantes est évitée.
